# EUROPÄISCHE PATENTSCHRIFT

(11) **EP 1 943 223 B1**
(45) Veröffentlichungstag und Bekanntmachung des Hinweises auf die Patenterteilung: **27.11.2013**
(21) Anmeldenummer: 06708555.5
(22) Anmeldetag: 28.02.2006
(51) Int. Cl.: C07D 219/12, A61K 31/473, C07D 401/12, C07D 417/12

(54) **9-AMINO-ACRIDIN-DERIVATE UND IHRE VERWENDUNG ZUR BESEITIGUNG FEHLGEFALTETER PROTEINE**
9-AMINO-ACRIDINE DERIVATIVES AND THEIR USE FOR ELIMINATING MISFOLDED PROTEINS
DERIVES DE 9-AMINO-ACRIDINE ET UTILISATION DANS L'ELIMINATION DE PROTEINES A MAUVAIS REPLIEMENT

(30) Priorität: 01.03.2005 DE 102005009909
(43) Veröffentlichungstag der Anmeldung: 16.07.2008
(73) Patentinhaber: Korth, Carsten, 40219 Düsseldorf (DE); Klingenstein, Ralf, 40724 Hilden (DE); Gmeiner, Dr. Peter, 91054 Erlangen-Buckenhof (DE); Löber, Stefan, 90607 Rückersdorf (DE)
(72) Erfinder: Korth, Carsten, 40219 Düsseldorf (DE); Klingenstein, Ralf, 40724 Hilden (DE); Gmeiner, Dr. Peter, 91054 Erlangen-Buckenhof (DE); Löber, Stefan, 90607 Rückersdorf (DE)
(74) Vertreter: Michalski Hüttermann & Partner Patentanwälte
(86) Internationale Anmeldenummer: PCT/EP2006/060328
(87) Internationale Veröffentlichungsnummer: WO 2006/092395

(56) Entgegenhaltungen:
- WO-A-2004/032929
- US-A1- 2004 229 898
- MAY B C H ET AL: "Potent inhibition of scrapie prion replication in cultured cells by bis-acridines" PROCEEDINGS OF THE NATIONAL ACADEMY OF SCIENCES OF USA, NATIONAL ACADEMY OF SCIENCE, WASHINGTON, DC, US, Bd. 100, Nr. 6, 18. März 2003 (2003-03-18), Seiten 3416-3421, XP002386930
- KOCISKO D A ET AL: "New Inhibitors of Scrapie-Associated Prion Protein Formation in a Library of 2,000 Drugs and Natural Products" JOURNAL OF VIROLOGY, THE AMERICAN SOCIETY FOR MICROBIOLOGY, US, Bd. 77, Nr. 19, Oktober 2003 (2003-10), Seiten 10288-10294, XP008053706 ISSN: 0022-538X
- KORTH C ET AL: "Acridine and phenothiazine derivatives as pharmacotherapeutics for prion disease" PROCEEDINGS OF THE NATIONAL ACADEMY OF SCIENCES OF USA, NATIONAL ACADEMY OF SCIENCE, WASHINGTON, DC, US, Bd. 98, Nr. 17, 14. August 2001 (2001-08-14), Seiten 9836-9841, XP002237386 ISSN: 0027-8424
- PRUSINER S B ET AL: "THERAPEUTIC APPROACHES TO PRION DISEASES" COLD SPRING HARBOR MONOGRAPH SERIES, COLD SPRING HARBOR LABORATORY, COLD SPRING HARBOR, NY, US, 2004, Seiten 961-1014, XP009065375 ISSN: 0270-1847

## Beschreibung

Die Erfindung betrifft Verbindungen, deren Enantiomere, Diastereomere sowie deren pharmazeutisch verträgliche Salze, deren Verwendung zur Herstellung eines Arzneimittels sowie Arzneimittel. Die Verbindungen sind geeignet zur Behandlung von Erkrankungen in Zusammenhang mit fehlgefalteten Proteinen.

Prionerkrankungen sind tödliche neurodegenerative Erkrankungen, die sowohl beim Menschen, beispielsweise Creutzfeld-Jakob-Erkrankung (CJD), als auch im Tierreich, beispielsweise bovine spongiforme Enzephalopathie (BSE) bei Rindern oder Scrapie bei Schafen, vorkommen können. Die Krankheiten können sowohl spontan auftreten, als auch durch Vererbung und durch Infektionen. Das infektiöse Agens ist das sogenannte Prion, das aus einer falsch gefalteten Form eines im Organismus vorkommendem Proteins, des Prionproteins, besteht. Im Falle einer Erkrankung wird dieses Prion im zentralen Nervensystem akkumuliert und führt dort zum Zelltod. Obwohl diese Krankheiten beim Menschen nur sehr selten vorkommen hat sich in den letzten Jahren der öffentliche Fokus auf sie gerichtet, da sich herausgestellt hat, dass bei Rindern vorkommendes BSE durch die Nahrungskette auf den Menschen übertragen werden kann und dort CJD auslösen kann.

Extrazelluläre oder intrazelluläre Proteinaggregationen sind auch ein phänotypisches Kennzeichen chronisch-degenerativer Erkrankungen, insbesondere neurodegenerativer und/oder neuropsychiatrischer Erkrankungen beispielsweise des Morbus Alzheimer, Morbus Parkinson oder Tauopathien.

Im Stand der Technik sind verschiedene Substanzen bekannt, die eine Wirksamkeit gegen Prionen zeigen. Nachteilig bei den bekannten Substanzen ist jedoch, dass diese aufgrund ihrer Polarität die Bluthirnschranke nicht überwinden können und somit nicht in das Gehirn gelangen können. Weiterhin sind viele der Substanzen in niedrigen Dosen nicht wirksam, zeigen jedoch bei höherer Dosierungen eine toxische Wirkung.

Die in der WO 02/096431 offenbarten Acridin- und Phenothiazinderivate weisen beispielsweise den Nachteil auf, dass sie in niedrigen Dosen nicht oder nur wenig wirksam sind. Weiterhin können Acridine, insbesondere bis-Acridine, toxisch wirken. Zudem zeigen diese Substanzen lediglich eine transiente Besserungen der Krankheitssymptome, jedoch keinen langfristigen therapeutischen Effekt Dies zeigt einen weiteren Nachteil der bekannten Substanzen auf, die keine Wirkung in Stadien der fortgeschrittenen bzw. klinischen Erkrankung zeigen.

Die US-A1 20041229898 offenbart cyclische Bis-Verbindongen zur Beseitigung fehlgefalteter Proteine.

Die Schrift "Potent inhibition of scrapie prion replication in cultured cells by bis-acridines" von Barnaby C. H. May et al.. Proc Natl Acad Sci USA 2003 100(6) S. 3416-3421 offenbart eine wirksame Inhibition der Replikation des Scrapi-Prions durch Bis-Acridine in Zellkultur.

Die Aufgabe der vorliegenden Erfindung bestand darin, Verbindungen zur Verfügung zu stellen, die wenigsten einen der Nachteile des Standes der Technik überwinden. Insbesondere bestand die Aufgabe der vorliegenden Erfindung darin, Verbindungen zur Verfügung zu stellen, die eine verbesserte Wirksamkeit gegenüber chronisch-degenerativen Erkrankungen aufweisen.

Diese Aufgabe wird gelöst durch Verbindungen gemäß der allgemeinen Formel (1) wie nachstehend angegeben und/oder deren Enantiomere, Diastereomere sowie deren pharmazeutisch verträgliche Salze: 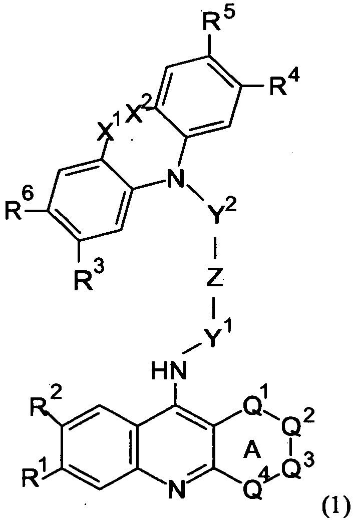 worin:
- A: ist ein sechsgliedriger, ungesättigter oder teilgesättigter Ring;
- Q¹, Q², Q³, Q⁴: sind jeweils unabhängig voneinander ausgewählt aus der Gruppe umfassend CH, C-Halogen, C-O-(C₁-C₁₀)-Alkyl, C-CF₃, C-CN und/oder CH₂;
- R¹, R², R³, R⁴, R⁵, R⁶: sind jeweils unabhängig voneinander ausgewählt aus der Gruppe umfassend H, Halogen, C₁-C₁₀-Alkyl, C₁-C₁₀-Alkenyl, C₁-C₁₀-Alkinyl; C₁-C₁₀-Alkyloxy, CF₃, NH₂, NHR⁹, wobei der Rest R⁹ ausgewählt ist aus der Gruppe umfassend C₁-C₁₀-Alkyl und/oder C₁-C₁₀-Acyl, NO₂ und/oder CN;
- X¹, X²: sind jeweils H, oder
X¹ und X² bilden gemeinsam X aus, wobei:
- X: ist ausgewählt aus der Gruppe umfassend CH₂, CH₂-CH₂, CH=CH und/oder O;
- Y¹, Y²: sind jeweils unabhängig voneinander unverzweigtes oder verzweigtes C₁-C₁₀-Alkyl;
- Z: ist ein Strukturelement Z1 oder Z2 wie nachstehend angegeben, wobei:
- Z1: weist die nachstehende allgemeine Formel (2) auf: 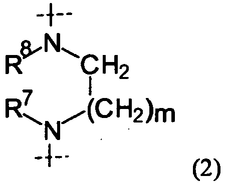 worin:
m ist 1, 2, 3, 4, 5 oder 6,
R⁷, R⁸ sind unabhängig voneinander ausgewählt aus der Gruppe umfassend H, C₁-C₁₀-Alkyl, wobei R⁷ und R⁸ gegebenenfalls über eine Gruppe CH₂-CH₂ einen Ring ausbilden, und/oder C₁-C₁₀-Acyl;
- Z2: weist die nachstehende allgemeine Formel (3) auf: 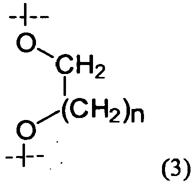 worin:
- n: ist 2, 3, 4, 5 oder 6.

Es wurde überraschend gefunden, dass die erfindungsgemäßen Verbindungen durch Zellmembranen hindurch gelangen können.

Ein besonderer Vorteil der Verbindungen ergibt sich hierbei dadurch, dass diese Verbindungen in niedrigeren Dosierungen eingesetzt werden können, wodurch die Verwendung der Verbindungen insbesondere bei Menschen ermöglicht wird.

Von besonderem Vorteil ist, dass die erfindungsgemäßen Verbindungen eine verminderte Toxizität aufweisen.

Es wurde weiterhin überraschend gefunden, dass die erfindungsgemäßen Verbindungen eine Fehlfaltung von Proteinen positiv beeinflussen können.

Insbesondere wurde überraschend gefunden, dass die sekundäre Aminogruppe in der Seitenkette der erfindungsgemäßen Verbindungen gemäß der Formel (1) die Wirksamkeit gegenüber einer tertiären Aminogruppe deutlich erhöhen kann.

Das Strukturelement A ist ein sechsgliedriger, ungesättigter oder teilgesättigter Ring, wobei A ein Kohlenstoffring ist. Q¹, Q², Q³ und Q⁴ umfassen entsprechend Kohlenstoffatome, die einen Kohlenstoffring ausbilden. Die Strukturelemente Q¹, Q², Q³ und Q⁴ können entsprechend der Sättigung des Rings A Gruppen CH oder CH₂ ausbilden.

Die Strukturelemente Q¹, Q², Q³ und Q⁴ sind jeweils unabhängig voneinander ausgewählt aus der Gruppe umfassend CH, C-Halogen. C-O-(C₁-C₁₀)-Alkyl, C-CF₃, C-CN und/oder CH₂. Bevorzugte Substituenten sind unabhängig voneinander ausgewählt aus der Gruppe umfassend H, Halogen ausgewählt aus der Gruppe umfassend F, Cl und/oder Br, und/oder C₁-C₅-Alkyloxy, vorzugsweise ausgewählt aus der Gruppe umfassend -O-Methyl, -O-Ethyl, -O-Isopropyl und/oder -O-tert-Butyl.

Die Strukturelemente Q¹, Q², Q³ und Q⁴ können entsprechend jeweils unabhängig voneinander ausgewählt sein aus der Gruppe umfassend CH, CH₂, C-Halogen, C-O-(C₁-C₁₀)-Alkyl, C-CF₃ und/oder C-CN. Bevorzugt sind die Strukturelemente Q¹, Q², Q³ und Q⁴ unabhängig voneinander ausgewählt aus der Gruppe umfassend CH, C-Halogen und/oder C-O-(C₁-C₅)-Alkyl, vorzugsweise ausgewählt aus der Gruppe umfassend C-O-Methyl, C-O-Ethyl, C-O-Isopropyl und/oder C-O-tert-Butyl. Besonders bevorzugt sind die Strukturelemente Q¹, Q², Q³ und Q⁴ unabhängig voneinander ausgewählt aus der Gruppe umfassend CH, C-Cl und/oder C-O-Methyl.

In bevorzugten Ausführungsformen ist das Strukturelement A ein sechsgliedriger, ungesättigter Kohlenstoffring. Weiterhin weist vorzugsweise das Strukturelement Q² Substituenten auf. Bevorzugt bilden die Strukturelemente Q¹, Q³ und Q⁴ jeweils eine Gruppe CH aus und das Strukturelement Q² ist ausgewählt aus der Gruppe umfassend CH und/oder C-O-CH₃. In besonders bevorzugten Ausführungsformen bilden die Strukturelemente Q¹, Q³ und Q⁴ jeweils eine Gruppe CH aus und das Strukturelement Q² ist C-O-CH₃.

In weiterhin bevorzugten Ausführungsformen ist das Strukturelement A ein sechsgliedriger, teilgesättigter Kohlenstoffring, besonders bevorzugt ein nicht substituierter sechsgliedriger, teilgesättigter Ring. In diesen Ausführungsformen bilden vorzugsweise alle Strukturelemente Q¹, Q², Q³ und Q⁴ jeweils eine Gruppe CH₂ aus.

Die Reste R¹, R², R³, R¹, R⁵ und R⁶ der erfindungsgeinäßen Verbindungen sind jeweils unabhängig voneinander ausgewählt aus der Gruppe umfassend H, Halogen, C₁-C₁₀-Alkyl, C₁-C₁₀-Alkenyl, C₁-C₁₀-Alkinyl, C₁-C₁₀-Alkyloxy, CF₃, NH₂, NHR⁹, wobei der Rest R⁹ ausgewählt ist aus der Gruppe umfassend C₁-C₁₀-Alkyl und/oder C₁-C₁₀-Acyl, NO₂ und/oder CN.

Bevorzugt sind die Reste R¹, R², R³, R⁴, R⁵ und R⁶ jeweils unabhängig voneinander ausgewählt aus der Gruppe umfassend H, Halogen ausgewählt aus der Gruppe umfassend F, Cl und/oder Br, NH₂, NHR⁹, wobei der Rest R⁹ ausgewählt ist aus der Gruppe umfassend C₁-C₁₀-Alkyl, bevorzugt C₁-C₅-Alkyl, vorzugsweise ausgewählt aus der Gruppe umfassend Methyl, Ethyl, Isopropyl und/oder tert-Butyl, und/oder C₁-C₁₀-Acyl, bevorzugt C₁-C₅-Acyl, NO₂, C₁-C₁₀-Alkyl, vorzugsweise ausgewählt aus der Gruppe umfassend Methyl, Ethyl, n-Propyl, Isopropyl, n-Butyl, tert.-Butyl, n-Pentyl, n-Hexyl, n-Heptyl und/oder n-Octyl, bevorzugt C₁-C₅-Alkyl, vorzugsweise ausgewählt aus der Gruppe umfassend Methyl, Ethyl, Isopropyl und/oder tert-Butyl, und/oder C₁-C₅-Alkyloxy, vorzugsweise ausgewählt aus der Gruppe umfassend -O-Methyl, -O-Ethyl, -O-Isopropyl und/oder -O-tert-Butyl. In bevorzugten Ausführungsformen der erfindungsgemäßen Verbindungen sind die Reste R¹, R², R³, R⁴, R⁵ und R⁶ jeweils unabhängig voneinander ausgewählt aus der Gruppe umfassend H, Cl und/oder -O-Methyl, besonders bevorzugt unabhängig voneinander ausgewählt aus der Gruppe umfassend H und/oder Cl.

Eine Substitution mit Gruppen R¹, R², R³, R⁴, R⁵ und R⁶, die jeweils unabhängig voneinander ausgewählt sind aus der Gruppe umfassend H und/oder Cl kann eine erheblich Steigerung der Wirksamkeit der Verbindung zur Folge haben.

Die Strukturelemente X¹ und X² können jeweils H sein oder gemeinsam ein Strukturelement X ausbilden. Ausführungsformen umfassend Strukturelemente X¹ und X², die jeweils H sind, können beispielsweise eine Teilstruktur gemäß nachstehender Formel (4) aufweisen:

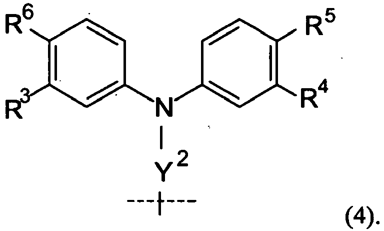

Bilden die Strukturelemente X¹ und X² gemeinsam ein Strukturelement X aus, ergibt sich eine Teilstruktur gemäß nachstehender Formel (5):

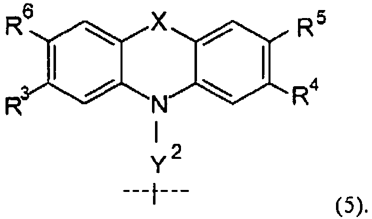

Das Strukturelemente X kann ein Heteroatom O sein, oder ausgewählt sein aus der Gruppe umfassend CH₂, CH₂-CH-, und/oder CH=CH. Somit ergeben sich Ringsysteme, die einen zentralen 6- oder 7-gliedrigen Ring aufweisen.

Besonders bevorzugte erfindungsgemäße Verbindungen weisen ein Strukturelement X ausgewählt aus der Gruppe umfassend -CH₂-CH₂- auf.

Vorzugsweise umfassen die erfindungsgemäßen Verbindungen ein Strukturelement Z1. Bevorzugte Ausführungsformen umfassend ein Strukturelement Z1 weisen die nachstehende Formel (6) auf:

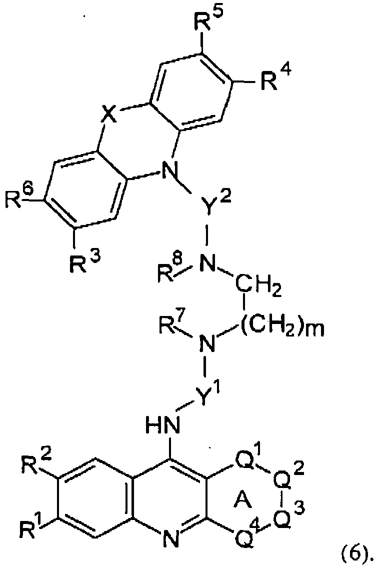

Die Reste R⁷ und R⁸ sind vorzugsweise I-I oder Alkylgruppen mit C₁ bis C₁₀ wie Methyl, Ethyl, n-Propyl, Isopropyl, n-Butyl, tert.-Butyl, n-Pentyl, n-Hexyl, n-Heptyl und/oder n-Octyl, vorzugsweise C₁-C₅-Alkyl, insbesondere ausgewählt aus der Gruppe umfassend Methyl, Ethyl, Isopropyl und/oder tert-Butyl. In diesen Ausführungsformen ist m 1, 2, 3, 4, 5 oder 6.

Vorzugsweise weist das Strukturelement Z1 eine ringförmige Struktur auf, wobei der Ringschluss über die Reste R⁷ und R⁸ erfolgt. Vorzugsweise erfolgt die Ringbildung dadurch, dass die Reste R⁷ und R⁸ jeweils eine Gruppe CH₂ ausbilden und gemeinsam eine Gruppe CH₁-CH₁ ausbilden. In diesen Ausführungsformen ist m vorzugsweise 1 oder 2, so dass ein heterocyclischer 6- oder 7-gliedriger Ring ausgebildet wird.

In bevorzugten Ausführungsformen ist m gleich 1 und das Strukturelement Z1 umfasst einen Piperazin-Heterocyclus. Das Strukturelement Z1 weist entsprechend in bevorzugten Ausführungsformen die nachstehende Formel (7) auf:

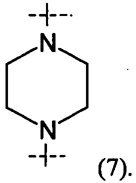

Weiterhin bevorzugte Ausführungsformen umfassend ein Strukturelement Z2 weisen die nachstehende Formel (8) auf, wobei n vorzugsweise 2, 3, 4 oder 5 ist:

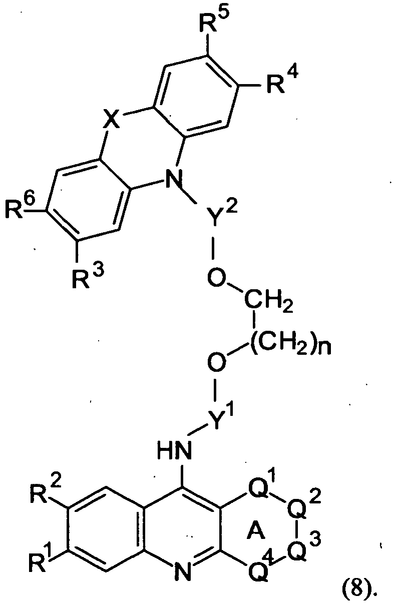

Die Strukturelemente Y¹, Y² sind jeweils unabhängig voneinander unverzweigtes oder verzweigtes C₁-C₁₀-Alkyl.

Besonders bevorzugte Verbindungen und/oder deren Enantiomere, Diastereomere sowie deren pharmazeutisch verträgliche Salze weisen die allgemeine Formel (9) auf, wie nachstehend angegeben: 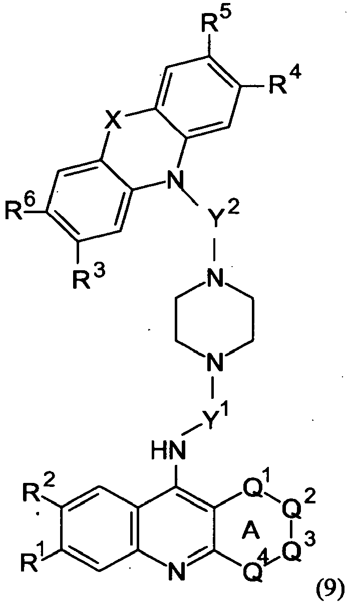 worin:
- A: ist ein sechsgliedriger, ungesättigter oder teilgesättigter Ring;
- Q¹, Q², Q³, Q⁴: sind jeweils unabhängig voneinander ausgewählt aus der Gruppe umfassend CH, C-Halogen, C-O-(C₁-C₁₀)-Alkyl, C-CF₃, C-CN und/oder CH₂;
- R², R², R³, R⁴ R⁵, R⁶: sind jeweils unabhängig voneinander ausgewählt aus der Gruppe umfassend H, Halogen, C₁-C₁₀-Alkyl, C₁-C₁₀-Alkenyl, C₁-C₁₀-Alkinyl, C₁-C₁₀-Alkyloxy, CF₃, NH₂, NHR⁹, wobei der Rest R⁹ ausgewählt ist aus der Gruppe umfassend C₁-C₁₀-ALkyl und/oder C₁-C₁₀-Acyl, NO₂ und/oder CN;
- X: ist ausgewählt aus der Gruppe umfassend CH₂, CH₂-CH₂, CH=CH, O und/oder S;
- Y¹, Y²: sind jeweils unabhängig voneinander unverzweigtes oder verzweigtes C₁-C₁₀-Alkyl.

Bevorzugt bilden die Strukturelemente Q¹, Q³ und Q⁴ der erfindungsgemäßen Verbindungen jeweils eine Gruppe CH aus und das Strukturelement, Q² ist ausgewählt aus der Gruppe umfassend CH und/oder C-O-CH₃. In besonders bevorzugten Ausführungsformen bilden die Strukturelemente Q¹ Q³ und Q⁴ jeweils eine Gruppe CH aus und das Strukturelement Q² ist C-O-CH₃. In weiterhin bevorzugten Ausführungsformen bilden vorzugsweise alle Strukturelemente Q¹, Q², Q³ und Q⁴ jeweils eine Gruppe CH₂ aus.

Bevorzugt sind die Reste R¹, R², R³, R⁴, R⁵ und R⁶ jeweils unabhängig voneinander ausgewählt aus der Gruppe umfassend H, Halogen ausgewählt aus der Gruppe umfassend F, Cl und/oder Br, NH₂, NIiR⁹, wobei der Rest R⁹ bevorzugt ausgewählt ist aus der Gruppe umfassend C₁-C₅-Alkyl, vorzugsweise ausgewählt aus der Gruppe umfassend Methyl, Ethyl, Isopropyl und/oder tert-Butyl, und/oder C₁-C₅-Acyl, NO₂, C₁-C₅-Alkyl, vorzugsweise ausgewählt aus der Gruppe umfassend Methyl, Methyl, Isopropyl und/oder tert-Butyl, und/oder C₁-C₅-Alkyloxy, vorzugsweise ausgewählt aus der Gruppe umfassend -O-Methyl, -O-Ethyl, - O-Isopropyl und/oder -O-tert-Butyl. In besonders bevorzugten Ausführungsformen der erfindungsgemäßen Verbindungen sind die Reste R¹, R², R³, R⁴, R⁵ und R⁶ jeweils unabhängig voneinander ausgewählt aus der Gruppe umfassend H, Cl und/oder -O-Methyl, besonders bevorzugt unabhängig voneinander ausgewählt aus der Gruppe umfassend H und/oder Cl.

Besonders bevorzugte Verbindungen weisen ein Strukturelement X ausgewählt aus der Gruppe umfassend -CH₂-CH₂- auf.

Die Strukturelemente Y¹ und Y² der erfindungsgemäßen Verbindungen können jeweils unabhängig voneinander wenigstens eine Verzweigung aufweisen, wobei Methyl- und/oder Ethyl-Seitenketten bevorzugt sind. Bevorzugt sind die Strukturelemente Y¹ und Y² jeweils unabhängig voneinander ausgewählt aus der Gruppe umfassend -(CH₂)ₒ-, wobei o ist 2, 3, 4, 5 oder 6, bevorzugt 3 oder 4, und/oder -CH(CH₃)-(CH₂)ₚ-, wobei p ist 2, 3, 4 oder 5, bevorzugt 3 oder 4, besonders bevorzugt 3. In ganz besonders bevorzugten Ausführungsformen sind die Strukturelemente Y¹ und Y² jeweils unabhängig voneinander -(CH₂)ₒ-, wobei o 3, 4 oder 5 ist.

In besonders bevorzugten Verbindungen weist der durch die Gruppe der Strukturelemente Y¹-Z-Y² gebildete Abstand zwischen den an die Strukturelemente Y¹ und Y² jeweils anschließenden Stickstoffatomen eine Länge von 10 Å bis 15 Å auf.

Besonders geeignete Verbindungen weisen Strukturelemente Q¹ Q³ und Q⁴ auf, die jeweils eine Gruppe CH ausbilden, und ein Strukturelement Q² ausgewählt aus der Gruppe umfassend CH und/oder C-O-CH₃, wobei das Strukturelement Q² vorzugsweise C-O-CH₃ ist, und Strukturelemente Y¹, Y², die jeweils unabhängig voneinander ausgewählt sind aus der Gruppe umfassend -(CH₂)ₒ- und/oder -CH(CH₃)-(CH₂)ₚ-, wobei o 2, 3, 4, 5 oder 6 ist und p ist 2, 3, 4 oder 5.

Ferner besonders geeignete Verbindungen weisen Strukturelemente Q¹ Q³ und Q⁴ auf, die jeweils eine Gruppe CH ausbilden, und ein Strukturelement Q² ausgewählt aus der Gruppe umfassend CH und/oder C-O-CH₃, wobei das Strukturelement Q² vorzugsweise C-O-CH₃ ist, und Strukturelemente Y¹, Y² , die jeweils unabhängig voneinander -(CH₂)ₒ- sind, wobei o 3, 4 oder 5 ist.

Ganz besonders geeignete Verbindungen umfassen Strukturelemente Q¹, Q³ und Q⁴, die jeweils eine Gruppe CH ausbilden, und ein Strukturelement Q² ausgewählt aus der Gruppe umfassend CH und/oder C-O-CH₃, wobei das Strukturelement Q² vorzugsweise C-O-CH₃ ist, und Strukturelemente Y¹, Y² die jeweils unabhängig voneinander -(CH₂)ₒ- sind, wobei o 3, 4 oder 5 ist und die Reste R¹, R², R³, R⁴, R⁵, R⁶ jeweils unabhängig voneinander ausgewählt aus der Gruppe umfassend H und/oder Cl sind.

Besonders bevorzugte Ausfuhrungsformen der erfindungsgemäßen Verbindungen weisen die nachstehend angegebene Formel (10) auf und/oder sind deren Enantiomere, Diastereomere sowie deren pharmazeutisch verträgliche Salze:

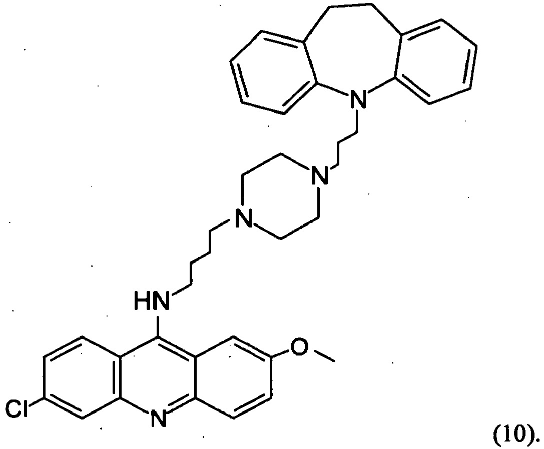

Weiterhin bevorzugte Ausführungsformen der erfindungsgemäßen Verbindungen weisen die nachstehend angegebene Formel (11) auf und/oder sind deren Enantiomere, Diastereomere sowie deren pharmazeutisch verträgliche Salze:

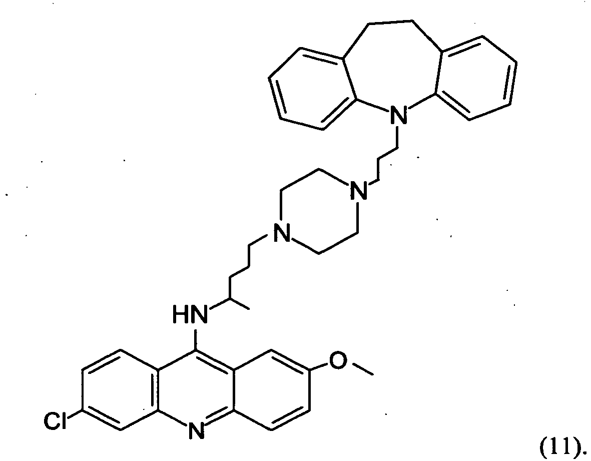

Beschrieben werden Verbindungen der nachstehend angegebene Formel (12) und/oder deren Enantiomere, Diastereomere. sowie deren pharmazeutisch verträgliche Salze:

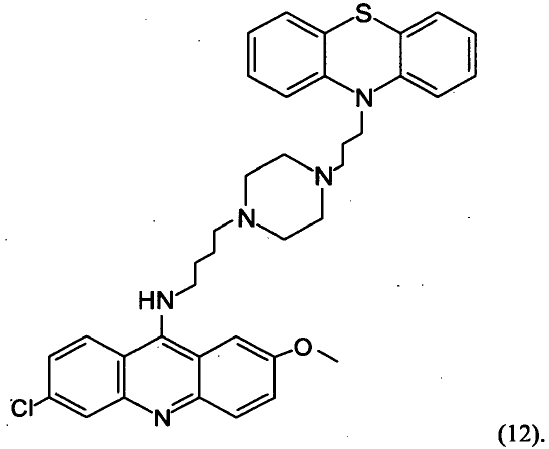

Ferner beschrieben werden Verbindungen der nachstehend angegebene Formel (13) und/oder deren Enantiomere, Diastereomere sowie deren pharmazeutisch verträgliche Salze:

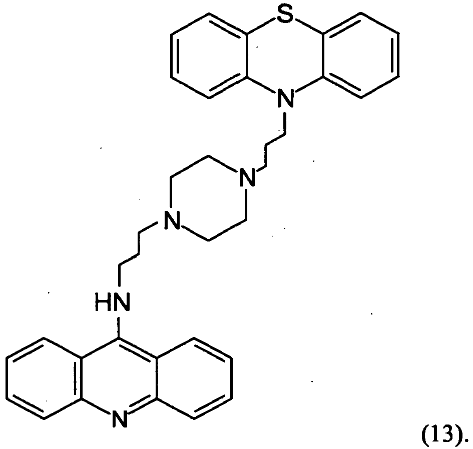

Noch weiter bevorzugte Ausführungsformen der erfindungsgemäßen Verbindungen weisen die nachstehend angegebene Formel (14) auf und/oder sind deren Enantiomere, Diastereomere sowie deren pharmazeutisch verträgliche Salze:

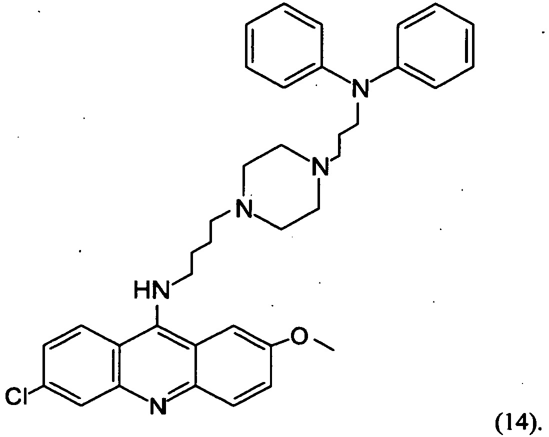

Bevorzugte Verbindungen sind ausgewählt aus der Gruppe umfassend (6-Chlor-2-methoxy-acridin-9-yl)-(4-{4-[3-(10,11-dihydro-dibenzo[b,f]azepin-5-yl)-propyl]-piperazin-1-yl}-butyl)-amin und/oder (6-Chlor-2-methoxy-acridin-9-yl)-(3-{4-[3-(dihydro-dibenzo[b,f]azepin-5-yl)-propyl]-piperazin-1-yl}-propyl)-amin, wobei die erstgenannte Verbindung der Verbindung (10) entspricht.

Weitere Ausführungsformen der erfindungsgemäßen Verbindungen weisen die nachstehend angegebenen Formeln (17) bis (21), (23) und (24) auf und/oder sind deren Enantiomere, Diastereomere sowie deren pharmazeutisch verträgliche Salze.

Beschrieben werden ferner Verbindungen der nachstehend angegebenen Formeln (15), (16) und (22) und/oder deren Enantiomere, Diastereomere sowie deren pharmazeutisch verträglichen Salze:

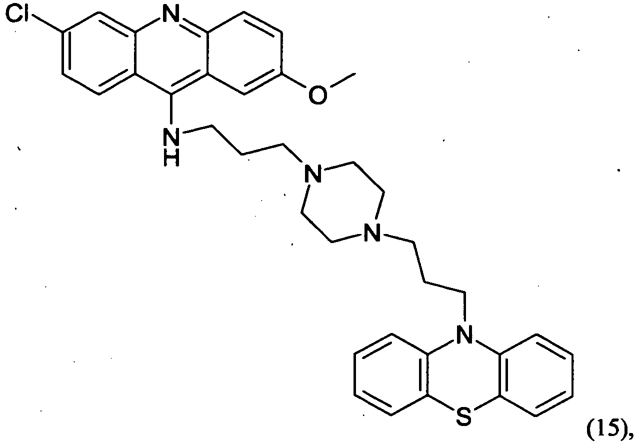

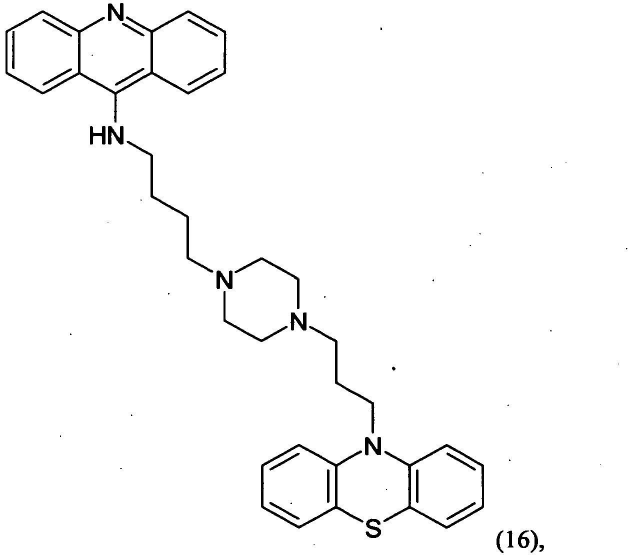

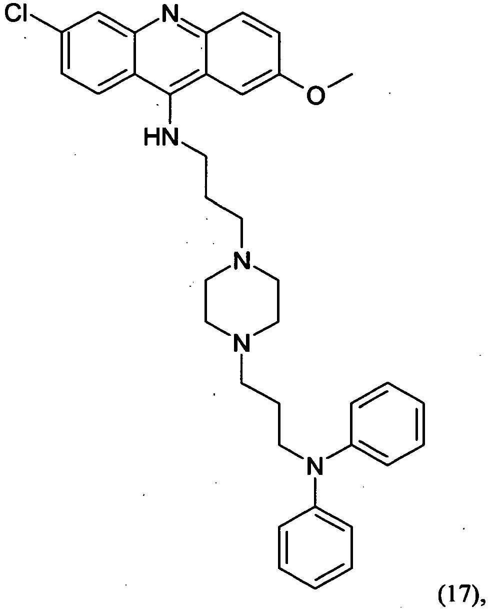

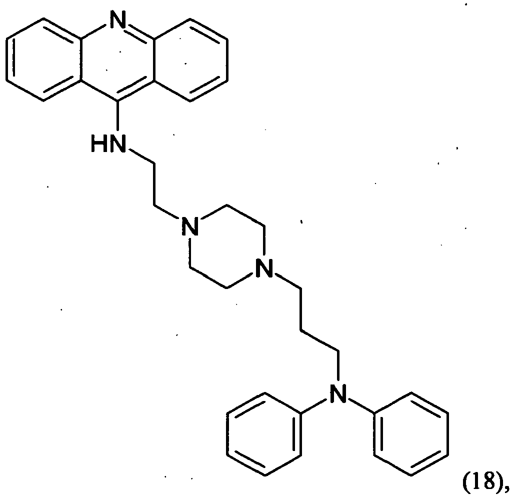

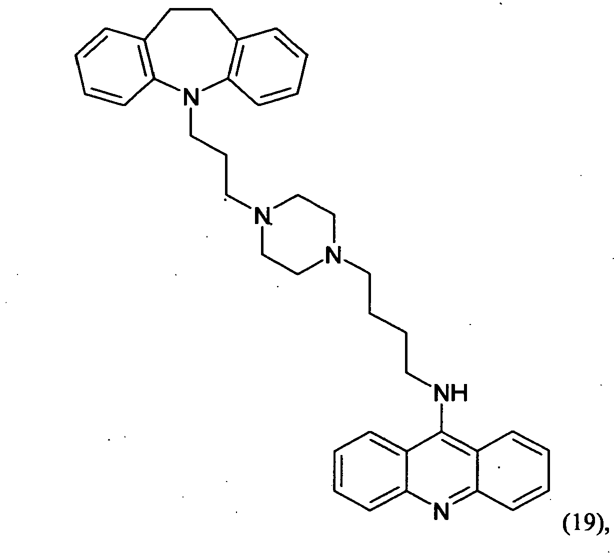

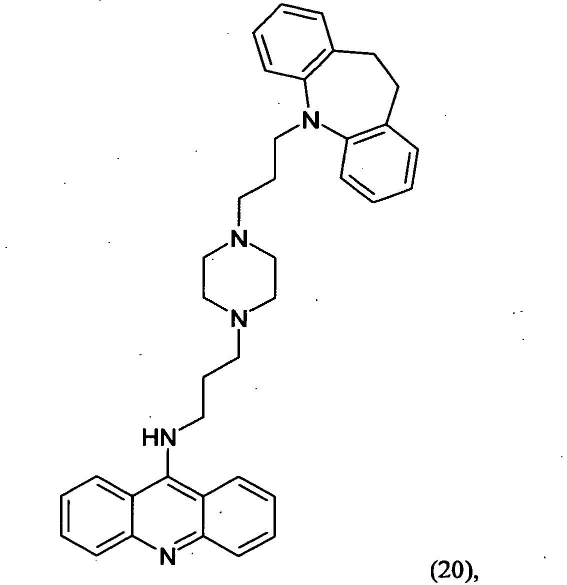

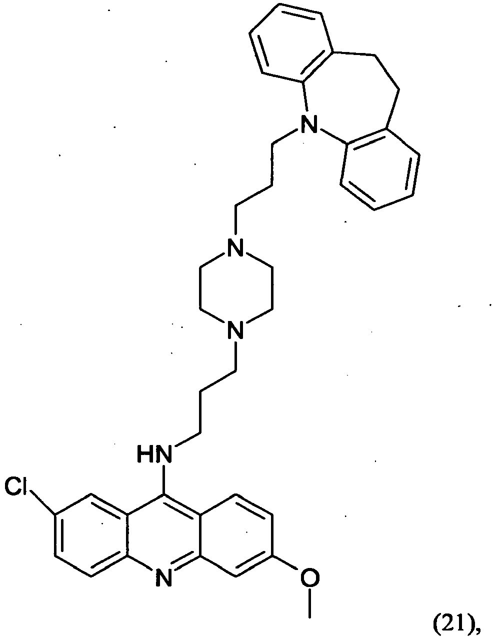

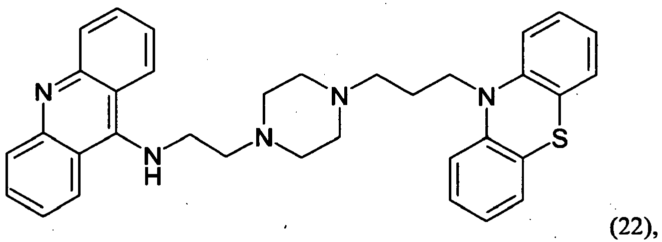

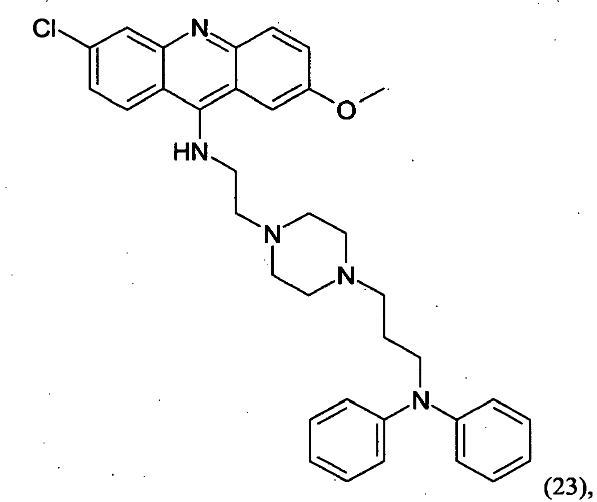

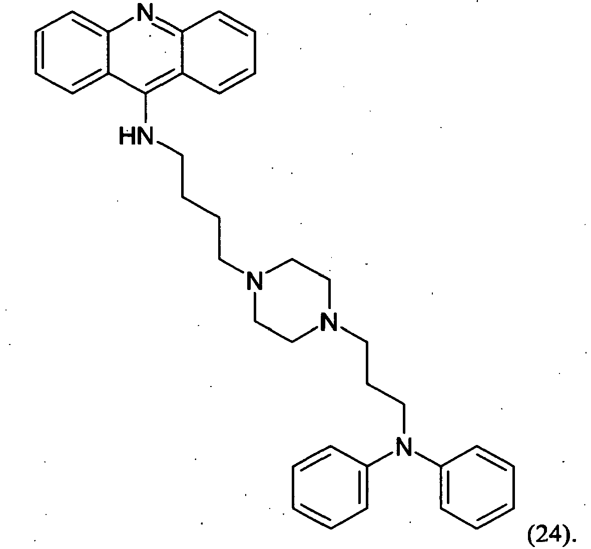

Die Verbindungen sind nach üblichen Synthesemethoden herstellbar

Ein Vorteil der Verbindungen liegt in bevorzugten Ausführungsformen darin, dass diese Verbindungen durch Zellmembranen hindurch gelangen können. Insbesondere können die erfindungsgemäßen Verbindungen eine gute Bluthirnschrankengängigkeit aufweisen. Dies ermöglicht, dass die erfindungsgemäßen Verbindungen in vivo und in vitro eingesetzt werden können. Erfindungsgemäße Verbindungen rind beispielsweise in Zellkulturen verwendbar. In besonders bevorzugten Ausführungsformen sind erfindungsgemäße Verbindungen auch in Geweben oder Organen verwendbar. Insbesondere ermöglichen die erfindungsgemäßen Verbindungen, in Organismen eingesetzt werden zu können.

Aufgrund ihrer vorteilhaften Eigenschaften sind die erfindungsgemäßen Verbindungen zur Verwendung als Arzneimittel geeignet.

Ein weiterer Gegenstand der Erfindung betrifft die Verwendung der erfindungsgemäßen Verbindungen, insbesondere der Verbindungen der allgemeinen Formeln (10) und (14), zur Herstellung eines Arzneimittels.

Die erfindungsgemäßen Verbindungen sind entsprechend üblichen Methoden verabreichbar, bevorzugt ist eine orale oder dermale Verabreichung und/oder eine Verabreichung mittels Injektion, beispielsweise intravenös, subkutan und/oder intramuskulär. Besonders bevorzugt ist eine orale Verabreichung.

Ein Vorteil der erfindungsgemäßen Verbindungen ist, dass diese eine verbesserte Bluthirnschrankengängigkeit aufweisen können. Hierdurch kann eine hohe Verfügbarkeit der Verbindungen an ihrem Wirkort, insbesondere in Zellen des Gehirns erzielt werden. Unter dem Begriff "Bluthirnschrankengängigkeit" wird im Sinne der Erfindung verstanden, dass die Verbindungen durch die Bluthirnschranke hindurchtreten und in das Gehirn, in die Zellen des Gehirns, insbesondere in Neurone, gelangen können. In besonders vorteilhaften Ausführungsformen gelangen die Verbindungen vollständig oder nahezu vollständig in die Zellen des Gehirns und/oder sind vollständig oder nahezu vollständig in den Zellen des Gehirns verfügbar.

Dies ist von besonderem Vorteil, da die Menge der zu verabreichenden Verbindungen bei den erfindungsgemäßen Verbindungen deutlich geringer sein kann, als bei Substanzen, die eine geringere Bluthirnschrankengängigkeit aufweisen. Vorteilhafter Weise kann entsprechend das Auftreten von Nebenwirkungen durch Verabreichung geringerer Dosierungen reduziert werden.

Bevorzugte Dosierungen der erfindungsgemäßen Verbindungen liegen für die Verabreichung an Menschen im Bereich von ≥1 mg/Tag/75 kg Körpergewicht bis ≤ 1000 mg/Tag/75 kg Körpergewicht, vorzugsweise im Bereich von ≥ 10 mg/Tag/75 kg Körpergewicht bis ≤ 500 mg/Tag/75 kg Körpergewicht, bevorzugt im Bereich von ≥ 30 mg/Tag/75 kg Körpergewicht bis ≤ 100 mg/Tag/75 kg Körpergewicht, besonders bevorzugt im Bereich von ≥ 30 mg/Tag/75 kg Körpergewicht bis ≤ 50 mg/Tag/75 kg Körpergewicht.

Die erfindungsgemäßen Verbindungen sind in vorteilhaften Ausführungsformen insbesondere zur therapeutischen und/oder prophylaktischen Behandlung, Diagnose und/oder Therapie von Erkrankungen, die mit einer Fehlfaltung von Proteinen in Zusammenhang stehen, verwendbar. Zu diesen Erkrankungen zählen beispielsweise Prionerkrankungen und/oder chronisch-degenerative Erkrankungen insbesondere neurodegenerative und/oder neuropsychiatrische Erkrankungen.

Unter dem Begriff "Fehlfaltung von Proteinen" wird im Sinne der Erfindung verstanden, dass Proteine fehlgefaltet oder missprozessiert sein können.

Vorteilhafter Weise sind die erfindungsgemäßen Verbindungen zur Behandlung von pflanzlichen und/oder tierischen Organismen, Geweben und/oder Zellen verwendbar. Insbesondere sind die erfindungsgemäßen Verbindungen zur Behandlung von Säugetieren beispielsweise des Menschen verwendbar.

Die erfindungsgemäßen Verbindungen können eine Fehlfaltung von Proteinen positiv beeinflussen. Insbesondere wurde überraschend gefunden, dass bevorzugte Ausführungsformen der Verbindungen die Menge vorhandener fehlgefalteter Prionproteine in Zellkulturmodellen verringern können. Vorteilhafter Weise können die erfindungsgemäßen Verbindungen in Experimenten eine höhere Wirksamkeit bei der Beeinflussung einer Fehlfaltung von Proteinen zeigen, als bislang bekannte Verbindungen. Insbesondere können die erfindungsgemäßen Verbindungen auch eine höhere Wirksamkeit zeigen, als Kombinationen der bislang bekannten Substanzen.

Ein besonderer Vorteil der erfindungsgemäßen Verbindungen kann dadurch verwirklicht werden, dass durch die Verabreichung der erfindungsgemäßen Verbindungen die Zeit von einer Infektion bis zum Ausbruch der Erkrankung verlängert werden kann und/oder die Überlebenszeit nach Ausbruch der Erkrankung verlängert werden kann.

Von besonderem Vorteil ist, dass die erfindungsgemäßen Verbindungen auch bei fortgeschrittenen bzw. klinischen Erkrankung zu einer Verminderung der fehlgefalteten Proteine, insbesondere Prionproteine führen können.

Von den erfindungsgemässen Verbindungen (e.V.) wurde experimentell festgestellt, dass sie die Cholesterolkonzentration auf der Zelloberfläche vermindern. Daher ergeben sich auch Anwendungen der e. V. auf Lipid/Cholesterolstoffwechselstörungen. Des weiteren ist von den Acridinen und Phenothiazinen eine anti-parasitäre / anti-Protozoenwirkung bekannt. Diese Wirkung wird von den e.V. ebenfalls erzielt und es ergeben sich daher Anwendungen in der Therapie von Protozoen und Parasitenerkrankungen. Insbesondere wird vermutet, dass die erfindungsgemäßen Verbindungen einen positiven Effekt in der Behandlung von Trypanosmen zeigen können.

Ohne auf eine bestimmte Theorie festgelegt zu sein, wird weiterhin vermutet, däss die erfindungsgemäßen Verbindungen einen positiven Effekt in der Behandlung und/oder Prophylaxe der Alzheimer Erkrankung haben können, und insbesondere einen positiven Effekt zur Verhinderung der Alzheimer Erkrankung zeigen können.

Entsprechend betrifft ein weiterer Gegenstand der Erfindung die Verwendung der erfindungsgemäßen Verbindungen, vorzugsweise der allgemeinen Formel (10) und (14), zur Herstellung eines Arzneimittels zur therapeutischen und/oder prophylaktischen Behandlung von Erkrankungen, ausgewählt aus der Gruppe umfassend:
- Erkrankungen, die mit einer Fehlfaltung von Proteinen in Zusammenhang stehen, wie Prionerkrankungen und/oder chronisch-degenerative Erkrankungen, insbesondere neurodegenerative und/oder neuropsychiatrische Erkrankungen,
- Erkrankungen, die mit einer Erhöhung des Cholesterols im Blut in Zusammenhang stehen,
- Erkrankungen, die mit einer Störung des Lipidstoffwechsels in Zusammenhang stehen und/oder Hyperlipidämien,
- Erkrankungen, die mit einer Entzündung in Zusammenhang stehen, wie chronische Entzündungen,
- kardiovaskuläre Erkrankungen insbesondere Arteriosklerose, und/oder
- durch Parasiten verursachte Infektionen bei Tieren und Pflanzen, insbesondere Menschen und Nutztieren, durch Protozoen und/oder durch Würmer verursachte Erkrankungen, insbesondere durch Parasiten und/oder Protozoen verursachte zerebrale Infektionen.

Die Prionerkrankungen können insbesondere ausgewählt sein aus der Gruppe umfassend Creutzfeldt-Jakob Erkrankung, Gerstmann-Sträussler-Scheinker Erkrankung, fatale familiäre Insomnie (FFI), Kuru, Scrapie und/oder bovine spongiforme Enzephalopathie (BSE).

Chronisch-degenerative Erkrankungen, insbesondere neurodegenerative und/oder neuropsychiatrische Erkrankungen, sind vorzugsweise ausgewählt aus der Gruppe umfassend Morbus Alzheimer, amyotrophe Lateralsklerose (ALS), Pick's Krankheit, Morbus Parkinson, multiple Sklerose, Morbus Huntington, Diabetes Typ I und Typ II, Autismus, Schizophrenie, bipolare Erkrankungen, Depression, Polyglutamin-Erkrankungen, frontotemporale Demenz, Tauopathien, multiple Systematropie, Schlafstörungen, Plasmazelldyskrasie, Familiäre Amyloide Polyneuropathie, medulläres Schilddrüsenkarzinom, chronische Niereninsuffizienz, kongestive Herzinsuffizienz, Amyloidose wie Herzamyloidose, systemische Amyloidose und/oder familiäre Amyloidose.

Bevorzugte neurodegenerative und/oder neuropsychiatrische Erkrankungen sind ausgewählt aus der Gruppe umfassend Morbus Alzheimer, frontotemporale Demenz, Morbus Parkinson, Tauopathien, multiple Systematropie, amyotrophe Lateralsklerose (ALS), Schizophrenie, bipolare Erkrankungen, Depression, Polyglutamin-Erkrankungen, multiple Sklerose und/oder Schlafstörungen.

Insbesondere bei der Behandlung von neurodegenerativen Erkrankungen des Menschen kann durch Verwendung der erfindungsgemäßen Verbindungen ein vorteilhafter Effekt auf den Krankheitsverlauf erzielt werden, insbesondere kann das Auftreten fehlgefalteter Proteine vermindert werden. Ein weiterer Vorteil der erfindungsgemäßen Verbindungen kann sich daraus ergeben, dass diese in niedriger Dosierung eingesetzt werden können. Weiterhin kann dies dazu führen, dass keine oder nur geringe Nebenwirkungen auftreten. Dies ermöglicht, dass die erfindungsgemäßen Verbindungen über einen längeren Zeitraum verabreicht werden können. Dies ermöglicht beispielsweise eine Verabreichung zur Behandlung von neurodegenerativen Erkrankungen, deren Therapie unter Umständen über Monate oder Jahre fortgesetzt werden muss.

In besonders vorteilhaften Ausführungsformen können die erfindungsgemäßen Verbindungen zu einer Erniedrigung des Cholesterols im Blut führen. Dies ermöglicht beispielsweise eine Verwendung bei Lipidstoffwechselstörungen oder Hyperlipidämien. Weiterhin können die Verbindungen auch antientzündliche Wirkung zeigen und zur therapeutischen und/oder prophylaktischen Behandlung von kardiovaskulären Erkrankungen verwendet werden, insbesondere zur Prophylaxe der Artheriosklerose.

Weiterhin sind die Verbindungen vorteilhafter Weise auch zur therapeutischen und/oder prophylaktischen Behandlung von Malaria und anderen Parasiten-verursachter Infektionen bei Lebewesen, Tiere und Pflanzen, insbesondere Nutztieren und Mensch, wie Malaria, Trypanosomiasis, Schlafkrankheit, Amoebenruhr, Leishmaniose und/oder Toxoplasmose. Auch eine Verwendung als Antiprotozoenmittel und/oder als Antihelminthika beispielsweise gegen Nematoden wie Madenwurm (Enterobius), Spulwurm (Ascaris), Hunde- u. Katzenspulwurm (Toxocara), Peitschenwurm (Trichuris), Hakenwurm (Nacator, Anchylostoma), Zwergfadenwurm (Spobngyloides), Drachenwurm (Dracunculus), oder Filarien, Cestoden, und/oder Trematoden, verwendbar.

Ein großer Vorteil bei der Verwendung der erfindungsgemäßen Verbindungen, vorzugsweise der Formel (10) kann dadurch verwirklicht werden, dass diese eine bessere Bluthirnschrankengängigkeit besitzen und insbesondere bei zerebralen Parasiten und/oder Protozoeninfektionen wie zerebrale Malaria, Toxoplasmose und/oder zerebralen Abzessen eine verbesserte Wirksamkeit zeigen können.

Ohne auf eine bestimmte Theorie festgelegt zu sein, wird weiterhin vermutet, dass die erfindungsgemäßen Verbindungen, vorzugsweise der Formel (10), einen hemmenden Effekt auf sog. Multidrug-Resistance (MDR) Proteine, wie das P- Glykoprotein, zeigen können, so dass eine im vergleich zu bekannten Substanzen effektivere Wirksamkeit und Bioverfügbarkeit zur Verfügung gestellt werden kann.

Vorteilhafter Weise können die erfindungsgemäßen Verbindungen eine nur geringe oder vemachlässigbare Toxizität bei der Verabreichung aufweisen. Dies ermöglicht beispielsweise die Verabreichung in hohen Dosierungen, insbesondere die einmalige und/oder mehrmalige, zeitlich begrenzte Verabreichung in Dosierungen im Bereich von ≥ 10 mg/Tag/75 kg Körpergewicht bis≤ 1000 mg/Tag/75 kg Körpergewicht, beispielsweise über einen Zeitraum von mindestens drei Monaten. Die geringe oder vernachlässigbare Toxizität der erfindungsgemäßen Verbindungen ermöglicht auch eine Verabreichung in einer sogenannten pulsatorischen Therapie. Hierbei können Dosierungen im Bereich von ≥ 10 mg/Tag/75 kg Körpergewicht bis ≤ 1000 mg/Tag/75 kg Körpergewicht beispielsweise für jeweils eine Woche verabreicht werden, vorzugsweise wenigstens zweimal für eine Woche, unterbrochen von einem Zeitraum ohne Verabreichung. Eine solche Verabreichung kann die positive Wirkung der Verbindungen auf fehlgefaltete Proteine weiter verbessern.

Ein weiterer Gegenstand der Erfindung betrifft Arzneimittel umfassend erfindungsgemäße Verbindungen, vorzugsweise umfassend Verbindungen der Formeln (10) und (14).

Arzneimittel umfassend erfindungsgemäße Verbindungen sind zur Behandlung in vivo und in vitro verwendbar. Eine bevorzugte Verwendung der Arzneimittel umfassend erfindungsgemäße Verbindungen sind die therapeutische und/oder prophylaktische Behandlung von Erkrankungen, die mit einer Fehlfaltung von Proteinen in Zusammenhang stehen, wie Prionerkrankungen und/oder und/oder chronisch-degenerative Erkrankungen, insbesondere neurodegenerative und/oder neuropsychiatrische Erkrankungen.

Ein weiterer Gegenstand der Erfindung betrifft Antiprionmittel umfassend erfindungsgemäße Verbindungen, vorzugsweise Verbindungen der Formel (10).

Der Begriff "Antiprionmittel" hat im Sinne dieser Erfindung die Bedeutung, dass das Mittel umfassend erfindungsgemäße Verbindungen Prionerkrankungen positiv beeinflussen kann. Insbesondere kann die Menge auftretender fehlgefalteter Prionproteine (PrP^{Sc}) vermindert werden.

In bevorzugten Ausführungsformen kann die Verwendung der erfindungsgemäßen Verbindungen dazu führen, dass in den untersuchten Medien, Zellen, Geweben und/oder Organen die Menge vorhandener fehlgefalteter Proteine, insbesondere Prionproteine (PrP^{Sc}) abnimmt und/oder ein Auftreten von fehlgefalteten Proteinen vollständig oder nahezu vollständig vermieden werden kann.

Die erfindungsgemäßen Verbindungen können beispielsweise in Experimenten zeigen, dass diese im Vergleich aktiver sind, als bekannte Substanzen oder auch deren Kombinationen. So können die erfindungsgemäßen Verbindungen dazu führen, dass die Menge vorhandener fehlgefalteter Prionproteine in höherem Ausmaß abnimmt, als bei einer Verabreichung einzelner bekannter Substanzen oder deren Kombination.

Beschrieben wird die Verwendung von erfindungsgemäßen Verbindungen vorzugsweise von Verbindungen er allgemeinen Formel (10), zur Reinigung von biologischen Flüssigkeiten, insbesondere Körperflüssigkeiten, die fehlgefaltete Proteine vorzugsweise Prionen enthalten, insbesondere von Zellsuspensionen. Die erfindungsgemäße Verbindungen können insbesondere zur Reinigung von mit fehlgefalteten Proteinen, vorzugsweise Prionen, kontaminierten biologischen Flüssigkeiten, verwendet werden.

Der Begriff "Reinigung" hat hier die Bedeutung, dass in den biologischen Flüssigkeiten, die Menge fehlgefalteter Proteine nach einer Zugabe der erfindungsgemäßen Verbindungen abnimmt, insbesondere kann der Begriff "Reinigung" im Sinne dieser Erfindung in der Bedeutung der Begriffe "Clearing" oder "Clearance" zu verstehen sein. Insbesondere können die erfindungsgemäßen Verbindungen in besonders vorteilhaften Ausführungsformen zu einer vollständigen oder nahezu vollständigen Beseitigung der fehlgefalteten Proteine führen.

Die Zellsuspensionen können beispielsweise Zerebrospinalflüssigkeit sein, vorzugsweise sind die Zellsuspensionen ausgewählt aus der Gruppe umfassend Blut und/oder oder Blutprodukte.

Hierbei können die Flüssigkeiten dem Organismus entnommen werden und die erfindungsgemäßen Verbindungen außerhalb des Organismus zugesetzt werden, oder die erfindungsgemäßen Verbindungen können den Flüssigkeiten zugesetzt werden, ohne dass diese dem Organismus entnommen werden.

Ferner wird ein Verfahren zur Reinigung von biologischen Flüssigkeiten, die fehlgefaltete Proteine, vorzugsweise Prionen, enthalten beschrieben, wobei erfindungsgemäße Verbindungen, vorzugsweise der Formel (10) und (14), der zu reinigenden biologischen Flüssigkeit, insbesondere Zellsuspensionen wie Blut oder Blutprodukten zugesetzt werden. Optional können die Verbindungen, beispielsweise durch Chromatographie, Dialyse und/oder Adsorption, nach einer Inkubation wieder aus der zu reinigenden biologischen Flüssigkeit abgetrennt werden.

In weiteren vorteilhaften Ausführungsformen des Verfahrens kann die biologischen Flüssigkeit vor und nach der Zugabe der erfindungsgemäße Verbindungen durch geeignete Methoden auf die Menge enthaltener fehlgefalteter Proteine untersucht werden.

In vorteilhaften Ausführungsformen des Verfahrens kann die zu reinigende biologische Flüssigkeit, insbesondere Zellsuspensionen wie Blut oder Blutprodukte, dem Organismus zunächst entnommen werden beispielsweise durch Punktion und nach der Reinigung dem Organismus wieder zugeführt werden, beispielsweise durch eine Plasmaferese.

In besonders vorteilhaften Ausführungsformen des Verfahrens zur Reinigung von biologischen Flüssigkeiten wie Zellsuspensionen, insbesondere Blut, umfasst dieses die nachstehenden Schritte:
a) optional Entnehmen der biologischen Flüssigkeit aus einem Organismus, beispielsweise durch Punktion;
b) Inkubieren der biologischen Flüssigkeiten mit den erfindungsgemäßen Verbindungen;
c) optional Abtrennen der Verbindungen, beispielsweise durch Chromatographie, Dialyse und/oder Adsorption;
d) optional wieder zufügen der biologischen Flüssigkeit, beispielsweise durch eine Plasmaferese.

Die Flüssigkeiten können alternativ beispielsweise in Form von Blutkonserven gelagert werden und/oder zu einem späteren Zeitpunkt dem Organismus, dem sie entnommen wurden oder einem anderen zugeführt werden.

Beispiele und Figuren, die der Veranschaulichung der vorliegenden Erfindung dienen, sind nachstehend angegeben.

In den Figuren zeigt:

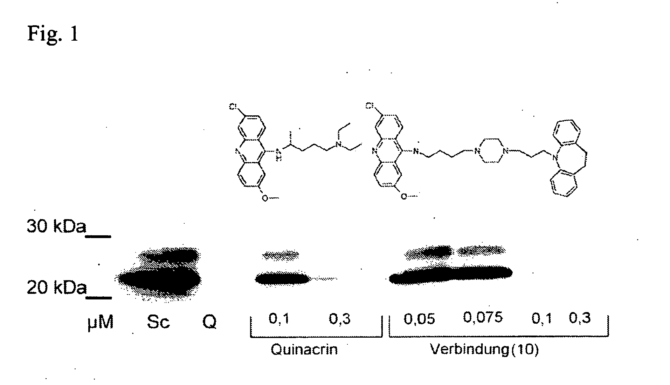

- Fig. 1: Western Blots von mit Protease K verdauten Zelllysaten von ScNa2-Zellen, die Banden des fehlgefalteten Prionproteins zeigen. Sc steht hierbei für die negative Kontrolle unbehandelter Zellen, die deutlich Banden des fehlgefalteten Prionproteins zeigen, Q steht für mit 1 µM Quinacrin behandelte Zellen als Positivkontrolle, die keine Banden des fehlgefalteten Prionproteins zeigen.
Es ist erkennbar, dass bei Zellkulturen, die mit einer Konzentration von 0,1 µM und 0,3 µM der Verbindung der Formel (10) inkubiert wurden, in den Lysaten keine fehlgefalteten Prionproteine nachweisbar waren, während die mit der identischen Konzentration Quinacrin behandelten Zellen noch fehlgefaltete Prionproteine enthielten.

### Beispiel 1

### Herstellung von (6-Chlor-2-methoxy-acridin-9-yl)-(4-{4-[3-(10,11-dihydro-dibenzo[b,f]azepin-5-yl)-propyl]-piperazin-1-yl}-butyl)-amin

Na(OAc)₃BH₃ (171 mg, 0,80 mmol) wurden zu einer Lösung von 3-(10,11-Dihydro-dibenzo[b,f]azepin-5-yl)-propionaldehyd (100 mg, 0,40 mmol) und N-(3-cyanopropyl)-piperazin (122 mg, 0,80 mmol) in CH₂Cl₂(10 ml) gegeben. Nach 16 Stunden Rühren wurde das Gemisch mit CH₂Cl₂ (50 ml) verdünnt und mit einer gesättigten Lösung von NaHCO₃ gewaschen. Die organische Phase wurde getrocknet (Na₂SO₄) und abgedampft. Reinigung mittels Flash-Chromatographie des erhaltenen Rückstands ergab N-[3-(10,11-Dihydro-dibenzo[b,f]azepin-5-yl)-propyl]-N'-(3-cyanopropyl)-piperazin (Ausbeute: 106 mg, 68%).

N-[3-(10,11-Dihydro-dibenzo[b,f]azepin-5-yl)-propyl]-N'-(3-cyanopropyl)-piperazin (60 mg 0,15 mmol) wurde in trockenem Diethylether (Et₂O) (5 ml) gelöst und mit LiAlH₄ (1,0 M in Et₂O, 0,15 ml, 0,15 mmol) bei 0°C versetzt. Nach einer Reaktionszeit von 3 Stunden wurden 3 Tropfen 0,5 N NaOH zugegen und das Gemisch wurde über Kieselgel (Celite^{®}, 521 AW) und MgSO₄ filtriert. Evaporation des Lösemittels ergab 48 mg N-[3-(10,11-Dihydro-dibenzo[b,f]azepin-5-yl)-propyl]-N'-(3-aminopropyl)-piperazin, das unter leichtem Erwärmen in Phenol (1,5 g) gelöst wurde. Zugabe von 6,9-Dichloro-2-methoxy-acridin (70 mg, 0,25 mmol) wurde von 1 Stunde Rühren bei 100°C gefolgt. Nachfolgende Zugabe von wässrigem NaOH, Extraktion mit EtOAc und verdampfen der organischen Phase ergab (6-Chloro-2-methoxy-acridin-9-yl)-(4-{4-[3-(10,11-dihydro-dibenzo[b,f]azepin-5-yl)-propyl]-piperazin-1-yl}-butyl)-amin, das durch Flash-Chromatographie gereinigt wurde, in einer Ausbeute von 21%.

### Beispiel 2

### Isolation und Reinigung eines polyklonalen Huhn-anti-Maus-Prionprotein-Antikörpers

### Immunglobulin-Isolation:

Eier von mit rekombinantem Maus-Prionprotein (recMoPrP) immunisierten Hühnern wurden gesammelt. Die Eidotter wurden im Verhältnis 1 : 5 in kalter 20 mM Natriumacetatlösung, pH 5,2, verdünnt und über Nacht bei 4°C aufbewahrt. Nach Entfernen von unlöslichem Material durch Zentrifugation (20.000 x g, 20 min) wurde das Immunglobulin durch Zugabe von 20% (w/v) (NH₄)₂SO₄ gefällt und nach 2 Stunden bei 4°C zentrifugiert (20.000 x g, 20 min). Das pelletierte Immunglobulin wurde in einer Lösung von 20 mM Tris pH 8, 150 mM NaCl, 0, 1 % Tween-20, 1 mM EDTA gelöst.

### Reinigung:

Rekombinantes Maus-Prionprotein (recMoPrP) wurde in einer Suspension von NHSaktivierter Sepharose (Amersham Pharmacia, vorgewaschen entsprechend den Anweisungen des Herstellerprotokolls) in einer kalten (4°C) Lösung von 50 mM NaHCO₃, pH 8,3, 1 % Triton X-100, 20% DMSO zu 3 mg Protein pro ml Harz gelöst. Diese Suspentsion wurde über Nacht bei 4°C gerührt. Freie NHS-Gruppen wurden anschließend mit 50 mM Glycin (1 Stunde, Raumtemperatur) blockiert. Das Harz wurde dann nacheinander mit Lösungen von 50 mM Tris pH 8, 50 mM Glycin pH 3, und abschließend 20 mM Tris pH 8, 150 NaCl, 0,2% Triton X100, 0,2% Tween-20, 2 mM EDTA, gewaschen. In diesem Puffer wurde immobilisiertes rekombinantes Maus-Prionprotein (recMoPrP) mit anti-Maus-Prionprotein-Immunglobulin (ungefähr 200 mg Immunglobulin pro mg Maus-Prionprotein) gemischt und über Nacht bei 4°C gerührt. Das Harz wurde dann gesammelt und ausführlich gewaschen, und nachfolgend mit einer Lösung von 100 mM Glycin pH 3, 1 M NaCl, 1 % Triton X-100 eluiert, wobei der pH-Wert sofort anschließend auf pH 8 eingestellt wurde.
Das gereinigte anti-Maus-Prionprotein-Immunglobulin wurde dann mit EZ-Link™ Maleimide aktivierter Meerettichperoxidase (HRP) (Pierce Chemical, Rockford, III.) entsprechend den Anweisungen des Herstellerprotokolls markiert.

Dieser Antikörper erkennt lediglich die nicht und monoglykosilierte Form des Prionproteins, die dem fehlgefalteten Prionprotein entspricht, jedoch nicht die diglykosilierte Form.

### Beispiel 3

### Zellkulturversuche mit Verbindungen der Formeln (12), (13) und (14)

Mausneuroblastomzellen (N2a) wurden mit mausadaptierten Scrapie-Prionen infiziert und subkloniert (Bosque, P. J. and S. B. Prusiner (2000) "Cultured cell sublines highly susceptible to prion infection" J Virol 74(9): 4377-86) und kultiviert. Konfluente 10-cm Zellkulturschalen wurden gesplittet, ein Tropfen mit 2-5*10⁴ der mit mausadaptierten Scrapie-Prionen infizierten Mausneuroblastomzellen (ScN2a) wurde jeweils in eine 60-mm Zellkulturschale gegeben und mit 4 ml MEM (Invitrogen, Carlsbad, USA) enthaltend 10% (vol/vol) fetales Kälberserum (FCS), Penicillin-Streptomycin (100 units/ml) und L-glutamin (2 mM) eine Woche mit 0,1 µM, 0,5µM und 1 µM der Verbindung (12) und (14) und 0,1 µM und 0,5µM der Verbindung (13) kultiviert. Während dieser Woche wurde an jedem 2. Tag das Medium gewechselt und die entsprechende Menge der getesteten Verbindung dem Medium zugesetzt.

Nach 7 Tagen wurden die Zellen einmal mit PBS (137 mM NaCl, 2,7 mM KCl, 10 mM Na₂HPO₄, 2 mM KH₂PO₄) gewaschen und in Lysispuffer (10 mM Tris, pH 8,0, 150 mM NaCl, 0,5% Triton X-100, 0,5% Deoxycholat) lysiert. Die Lysate wurden anschließend mit Proteinase K (Merck, Darmstadt) verdaut (20 µg/ml, 30 min, 37 °C) und die Reaktion mit 2 mM PMSF (Phenylmethylsulfonylfluorid)gestoppt. Anschließend wurden die verdauten Lysate zentrifugiert (45 min, 4°C, 100.000 g), der Überstand abgenommen und die Pellets in Gelbeladungspuffer (100 mM Tris-Cl pH 6,8, 4% (w/v) SDS, 0,2 % (w/v) Bromphenolblau, 20% (v/v) Glycerol, 200 mM β-Mercaptoethanol) aufgenommen. Die Proben werden über ein SDS-Gel (4-20%, Biorad) aufgetrennt und das Prionprotein mit 0,3 µg/ml des Antikörpers gemäß Beispiel 2 mittels Western Blotting detektiert (Enhanced Chemiluminescence (ECL) System, Amersham Pharmacia).

Als negative Kontrolle dienten mit Mausadaptierten Scrapie-Prionen infizierte Mausneuroblastomzellen, die nicht mit Quinacrin behandelt wurden, als positive Kontrolle dienten Zellen, die unter Versuchsbedingungen mit 1 µM der bekannten Antiprionsubstanz Quinacrin behandelt wurden.

Die fehlgefaltete Form des Prionproteins wurde durch die Behandlung mit Proteinase K nicht zerstört und war im Western Blot nachweisbar.

Es zeigte sich, dass die Verbindungen (12), (13) und (14) eine Beseitigung des fehlgefalteten Prionproteins in den infizierten Zellen zeigten, wobei die Verbindungen (12) und (13) eine höhere Wirksamkeit als die Verbindung (14) zeigten.

### Beispiel 4

Entsprechend den Bedingungen von Beispiel 3 wurden mit Mausadaptierten Scrapie-Prionen infizierte Mausneuroblastomzellen mit 0,05 µM, 0,075 µM, 0,1 µM und 0,3 µM der Verbindung der Formel (10) inkubiert und aufgearbeitet und die fehlgefalteten Prionproteine im Western Blot nachgewiesen.

Als negative Kontrolle dienten mit Mausadaptierten Scrapie-Prionen infizierte Mausneuroblastomzellen, die nicht mit Quinacrin behandelt wurden, als positive Kontrolle dienten Zellen, die unter Versuchsbedingungen mit 1 µM der bekannten Antiprionsubstanz Quinacrin behandelt wurden. Als Vergleich dienten Zellen, die mit 0,1 µM und 0,3 µM Quinacrin behandelt wurden.

Es zeigte sich, dass bei Zellkulturen, die mit einer Konzentration von 0,1 µM und 0,3 µM der Verbindung der Formel (10) inkubiert wurden, in den Lysaten keine fehlgefalteten Prionproteine nachweisbar waren, während die mit der identischen Konzentration Quinacrin behandelten Zellen noch fehlgefaltete Prionproteine enthielten. Die Verbindung (10) zeigte sich ca. 10mal aktiver als Quinacrin.

Der in Fig. 1 dargestellte Western Blot dient der Verdeutlichung der Versuchsergebnisse des Beispiels 4.

### Beispiel 5

### Filipinfärbung

Die Zellen wurden auf Deckgläschen herangezogen und wie in den Beispielen 3 und 4 beschrieben mit 0,25 µM Verbindung (10) und 1 µM Quinacrin behandelt. Am sechsten Tag der Behandlung wurden die Zellen einmal mit eiskaltem PBS (137 mM NaCl, 2,7 mM KCl, 10 mM Na₂HPO₄, 2 mM KH₂PO₄) gewaschen. Anschließend folgte die Fixierung der Zellen mit 4% (w/v) Paraformaldehyd in PBS für 30 min bei Raumtemperatur. Danach wurde die Paraformaldehydlösung abgenommen und die Zellen mit Filipin (50µg/ml in PBS, Sigma) für 30 min bei Raumtemperatur inkubiert. Die Deckgläschen wurden 4 mal mit PBS gewaschen und unter dem Mikrospkop mit den entsprechenden Filtersätzen untersucht. Die Filipinfärbung detektiert zelluläres Cholesterol.

Es zeigte sich, dass bei unbehandelten Zellen (ScN2a) das Cholesterol überwiegend an der Zelloberfläche zu finden ist. Bei mit der Verbindung (10) behandelten Zellen zeigte sich eine Umverteilung zu intrazellulären Kompartimenten, ebenso wie bei den mit Quinacrin behandelten Zellen. Dies zeigt die Wirkung der Verbindungen auf Cholesterol.

### Beispiel 6

### Aktivitätsuntersuchungen in vivo an einem Mausmodell

Der Effekt der Verbindung gemäß der Formel (10) auf eine Prioninfektion in vivo wurde in einem Mausmodell der Prionerkrankungen untersucht.

Verwendet wurden transgene Mäuse (TG 20, Fischer et al., 1996, EMBO Journal Vol. 15, S. 1255-64), die zusätzlich zum endogenen Maus PrP-Gen das endogene Maus Protein PrP überexprimieren. Diese Mäuse haben eine verkürzte Inkubationszeit von Prionen von 92 ± 4 Tagen, weil sie aufgrund der hohen Konzentration von Prionprotein (PrP^{C}) im Gehirn besonders schnell Prionen vermehren, so dass eine Wirkung gegen diese beschleunigte Konversion notwendig ist.

Diese Mäuse wurden drei Wochen vor Beginn der Untersuchungen intrazerebral mit 100 µl eines 10⁻⁵ verdünnten RML-Scrapie-haltigen Hirnhomogenats (Chandler, Lancet, 1961, Vol. 1, S. 1378-79) inokuliert. Zu Beginn der Untersuchungen wird somit bereits eine positive Wirkung erschwert, weil sich bereits neurotoxische Wirkungen und/oder subklinische Hirnschädigungen ergeben haben können.

Die Verbindung gemäß der Formel (10) wurde zunächst in Ethanol gelöst und dann in Distelöl in eine Endkonzentration von 10mg/ml verdünnt. Die Verbindung wurde dann per Magensonde ("Gavage") einer Gruppe von 11 Mäusen dreimal wöchentlich (Montag/ Mittwoch/ Freitag) für acht Wochen in einer Dosis von 2mg/200µl/Maus appliziert.

Eine Kontrollgruppe von Mäusen, die nicht mit der Verbindung gemäß der Formel (10) behandelt wurden, starben entsprechend nach 92 ± 4 Tagen an der Prionerkrankung, während die Mäuse, die mit der Verbindung gemäß der Formel (10) behandelt wurden, erst nach 100 ± 5 Tagen starben.

Somit konnte eine Verlängerung der Überlebenszeit von 10 %, ermittelt mittels Student's t test (p <0.0001), unter den Versuchsbedingungen festgestellt werden. Diese Bedingungen kommen der klinischen Situation nahe, bei der Patienten mit der Creutzfeldt Jakoberkrankung erst im Spätstadium ärztliche Hilfe suchen.

## Patentansprüche

1. Verbindungen gemäß der allgemeinen Formel (1) wie nachstehend angegeben und/oder deren Enantiomere, Diastercomere sowie deren pharmazeutisch verträgliche Salze: 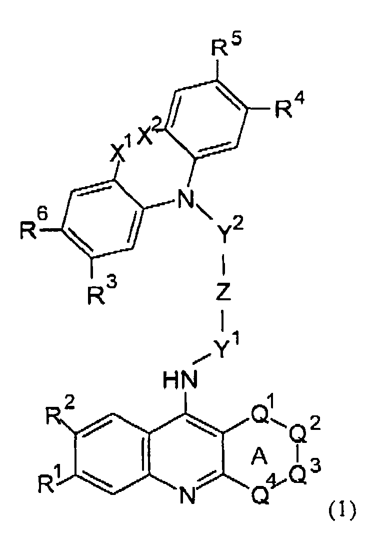 worin:
A ist ein sechsgliedriger, ungesättigter oder teilgesättigter Ring;
Q¹, Q², Q³, Q⁴ sind jeweils unabhängig voneinander ausgewählt aus der Gruppe umfassend CH, C-Halogen, C-O-(C₁-C₁₀)-Alkyl, C-CF₃, C-CN und/oder CH₂;
R¹, R², R³, R⁴, R⁵, R⁶ sind jeweils unabhängig voneinander ausgewählt aus der Gruppe umfassend H, Halogen, C₁-C₁₀-Alkyl, C₁-C₁₀-Alkenyl, C₁-C₁₀-Alkinyl, C₁-C₁₀-Alkyloxy, CF₃, NH₂, NHR⁹, wobei der Rest R⁹ ausgewählt ist aus der Gruppe umfassend C₁-C₁₀-Alkyl und/oder C₁-C₁₀-Acyl, NO₂ und/oder CN;
X¹, X² sind jeweils H, oder
X¹ und X² bilden gemeinsam X aus, wobei:
X ist ausgewählt aus der Gruppe umfassend CH₂, CH₂-CH₂, CH=CH und/oder O;
Y¹, Y² sind jeweils unabhängig voneinander unverzweigtes oder verzweigtes C₁-C₁₀-Alkyl;
Z ist ein Strukturelement Z1 oder Z2 wie nachstehend angegeben, wobei:
Z1 weist die nachstehende allgemeine Formel (2) auf: 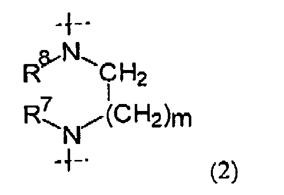 worin:
m ist 1, 2, 3, 4, 5 oder 6,
R⁷, R⁸ sind unabhängig voneinander ausgewählt aus der Gruppe umfassend H, C₁-C₁₀-Alkyl, wobei R⁷ und R⁸ gegebenenfalls über eine Gruppe CH₂-CH₂ einen Ring ausbilden, und/oder C₁-C₁₀-Acyl;
Z2 weist die nachstehende allgemeine Formel (3) auf: 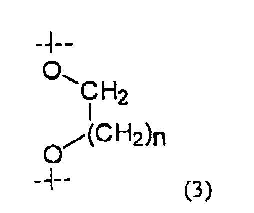 worin:
n ist 2, 3, 4, 5 oder 6.

2. Verbindungen nach Anspruch 1, **dadurch gekennzeichnet, dass** Z1 die nachstehende allgemeine Formel (7) aufweist:

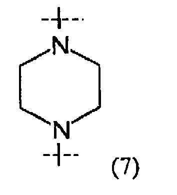

3. Verbindungen nach Anspruch 1 oder 2, **dadurch gekennzeichnet, dass** die Verbindungen die allgemeine Formel (9) wie nachstehend angegeben aufweisen und/oder deren Enantiomcre, Diastereomere sowie deren pharmazeutisch verträgliche Salze:

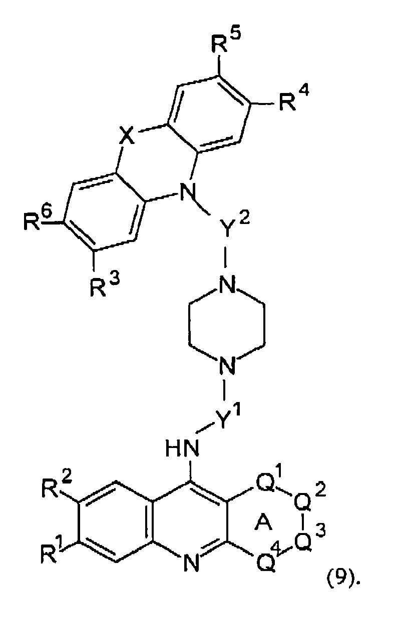

4. Verbindungen nach einem der vorherigen Ansprüche, **dadurch gekennzeichnet, dass**
Q¹, Q³, Q⁴ sind jeweils CH und
Q² ist ausgewählt aus der Gruppe umfassend CH und/oder C-O-CH₃.

5. Verbindungen nach einem der vorherigen Ansprüche, **dadurch gekennzeichnet, dass**
Q¹, Q², Q³, Q⁴ sind jeweils CH₂.

6. Verbindungen nach einem der vorherigen Ansprüche, **dadurch gekennzeichnet, dass**
Y¹, Y² sind jeweils unabhängig voneinander ausgewählt aus der Gruppe umfassend -(CH₂)ₒ- und/oder -CH(CH₃)-(CH₂)ₚ-, wobei:
o ist 2, 3, 4, 5 oder 6;
p ist 2, 3, 4 oder 5.

7. Verbindungen nach einem der vorherigen Ansprüche, **dadurch gekennzeichnet, dass**
R¹, R², R³, R⁴, R⁵, R⁶ sind jeweils unabhängig voneinander ausgewählt aus der Gruppe umfassend H, Halogen, vorzugsweise ausgewählt aus der Gruppe umfassend F, Cl und/oder Br, NH₂, NHR⁹, wobei der Rest R⁹ ausgewählt ist aus der Gruppe umfassend C₁-C₅-Alkyl und/oder C₁-C₅-Acyl, NO₂, C₁-C₅-Alkyl, vorzugsweise ausgewählt aus der Gruppe umfassend Methyl, Ethyl, Isopropyl und/oder tert-Butyl, und/oder C₁-C₅-Alkyloxy, vorzugsweise ausgewählt aus der Gruppe umfassend -O-Methyl, -O-Ethyl, -O-lsopropyl und/oder -O-tert-Butyl.

8. Verbindungen nach einem der vorherigen Ansprüche, **dadurch gekennzeichnet, dass**
X ist -CH₂-CH₂-.

9. Verbindungen nach Anspruch 4, **dadurch gekennzeichnet, dass**
Y¹, Y² sind jeweils unabhängig voneinander ausgewählt aus der Gruppe umfassend -(CH₂)ₒ- und/oder -CH(CH₃)-(CH₂)p-, wobei:
o ist 2, 3, 4, 5 oder 6;
p ist 2, 3, 4 oder 5.

10. Verbindungen nach Anspruch 4, **dadurch gekennzeichnet, dass**
Y¹, Y² sind jeweils unabhängig voneinander -(CH₂)ₒ-, wobei:
o ist 3, 4 oder 5.

11. Verbindungen nach Anspruch 10, **dadurch gekennzeichnet, dass**
R¹, R², R³, R⁴, R⁵, R⁶ sind jeweils unabhängig voneinander ausgewählt aus der Gruppe umfassend H und/oder Cl.

12. Verbindung nach einem der vorherigen Ansprüche, **gekennzeichnet durch** die nachstehend angegebene Formel (10) und/oder deren Enantiomere, Diastereomere sowie deren pharmazeutisch verträgliche Salze:

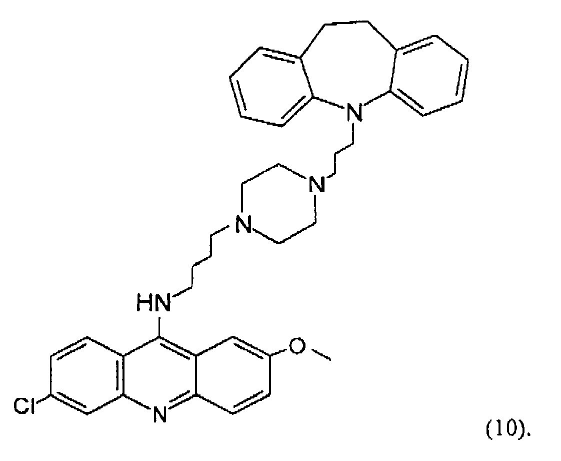

13. Verbindung nach einem der vorherigen Ansprüche, **gekennzeichnet durch** die nachstehend angegebene Formel (14) und/oder deren Enantiomere, Diastereomere sowie deren pharmazeutisch verträgliche Salze:

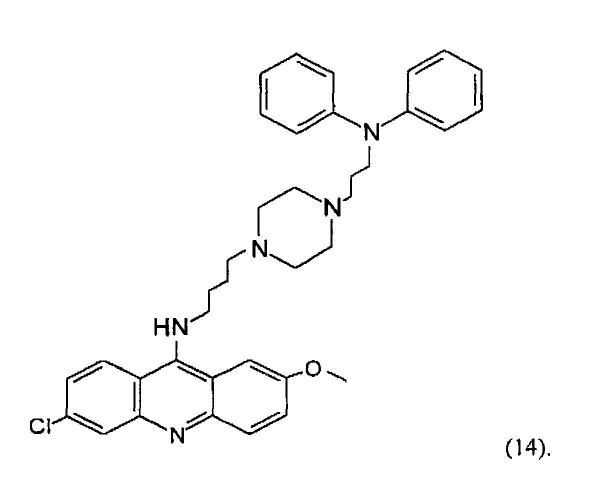

14. Verwendung von Verbindungen nach einem der vorherigen Ansprüche zur Herstellung eines Arzneimittels.

15. Verwendung von Verbindungen nach einem der vorherigen Ansprüche, vorzugsweise der Formel (10), zur Herstellung eines Arzneimittels zur therapeutischen und/oder prophylaktischen Behandlung von Erkrankungen ausgewählt aus der Gruppe umfassend:
- Erkrankungen, die mit einer Fehlfaltung von Proteinen in Zusammenhang stehen, wie Prionerkrankungen und/oder chronisch-degenerative Erkrankungen, insbesondere neurodegenerative und/oder neuropsychiatrische Erkrankungen,
- Erkrankungen, die mit einer Erhöhung des Cholesterols im Blut in Zusammenhang stehen,
- Erkrankungen, die mit einer Störung des Lipidstoffwechsels in Zusammenhang stehen und/oder Hyperlipidärnien,
- Erkrankungen, die mit einer Entzündung in Zusammenhang stehen, wie chronische Entzündungen,
- kardiovaskuläre Erkrankungen insbesondere Arteriosklerose, und/oder
- durch Parasiten verursachte Infektionen bei Tieren und Pflanzen, insbesondere Menschen und Nutztieren, durch Protozoen und/oder durch Würmer verursachte Erkrankungen, insbesondere durch Parasiten und/oder Protozoen verursachte zerebrale Infektionen.

16. Verwendung von Verbindungen nach Anspruch 15, **dadurch gekennzeichnet, dass** die Prionerkrankungen ausgewählt sind aus der Gruppe umfassend Creutzfeldt-Jakob Erkrankung, Gerstmann-Sträussler-Scheinker Erkrankung, fatale familiäre Insomnie, Kuru, Scrapie und/oder bovine spongiforme Enzephalopathie.

17. Verwendung von Verbindungen nach Anspruch 15, **dadurch gekennzeichnet, dass** die chronisch-degenerativen Erkrankungen insbesondere neurodegenerative und/oder neuropsychiatrische Erkrankungen ausgewählt sind aus der Gruppe umfassend Morbus Alzheimer, amyotrophe Lateralsklerose (ALS), Pick's Krankheit, Morbus Parkinson, multiple Sklerose, Morbus Huntington, Diabetes Typ I und Typ II, Autismus, Schizophrenie, bipolare Erkrankungen, Depression, Polyglutamin-Erkrankungen, frontotemporale Demenz, Tauopathien, multiple Systematropie, Schlafstörungen, Plasmazelldyskrasic, Familiäre Amyloide Polyneuropathie, medulläres Schilddrüscnkarzinom, chronische Niereninsuffizienz, kongestive Herzinsuffizienz, Amyloidose wie Herzamyloidose, systemische Amyloidose und/oder familiäre Amyloidose.

18. Verwendung von Verbindungen nach Anspruch 15, **dadurch gekennzeichnet, dass** die durch Parasiten verursachte Infektionen ausgewählt sind aus der Gruppe umfassend Malaria, Trypanosomiasis, Schlafkrankheit, Amoebenruhr, Leishmaniose und/oder Toxoplasmose.

19. Arzneimittel umfassend Verbindungen nach einem der vorherigen Ansprüche, vorzugsweise der Formel (10).

20. Antiprionmittel umfassend Verbindungen nach einem der vorherigen Ansprüche, vorzugsweise der Formel (10).

## Claims

1. Compounds of the general formula (1) as shown below and/or their enantiomers, diastereomers and their pharmaceutically acceptable salts: 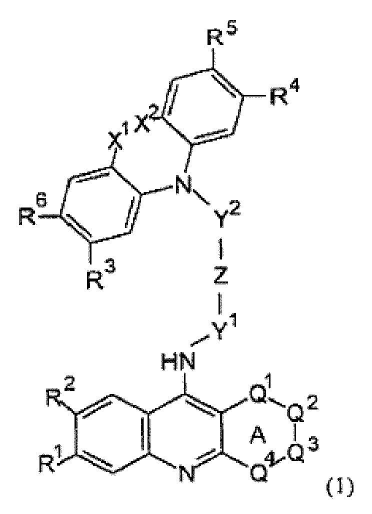 wherein:
A is a six-membered unsaturated or partially saturated ring;
Q¹, Q², Q³, Q⁴ are each independently of one another selected from the group comprising CH, C-halogen, C-O-(C₁-C₁₀)-alkyl, C-CF₃, C-CN and/or CH₂;
R¹, R², R³, R⁴, R⁵, R⁶ are each independently of one another selected from the group comprising H, halogen, C₁-C₁₀-alkyl, C₁-C₁₀-alkenyl, C₁-C₁₀-alkynyl, C₁-C₁₀-alkyloxy, CF₃, NH₂, NHR⁹, wherein the radical R⁹ is selected from the group comprising C₁-C₁₀-alkyl and/or C₁-C₁₀-acyl, NO₂ and/or CN;
X¹, X² are each H, or
X¹ and X² together form X, wherein:
X is selected from the group comprising CH₂, CH₂-CH₂, CH=CH and/or 0;
Y¹, Y² are each independently of one another unbranched or branched C₁-C₁₀-alkyl;
Z is a structural element Z1 or Z2 as shown below, wherein:
Z1 has the general formula (2) below: 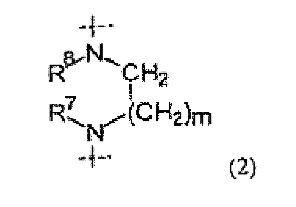 wherein:
m is 1, 2, 3, 4, 5 or 6,
R⁷, R⁸ are independently of one another selected from the group comprising H, C₁-C₁₀-alkyl, wherein R⁷ and R⁸ optionally, via a group CH₂-CH₂, form a ring, and/or C₁-C₁₀-acyl;
Z2 has the general formula (3) below: 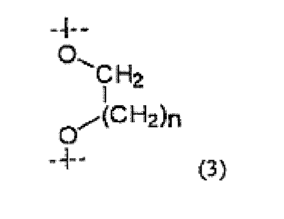 wherein:
n is 2, 3, 4, 5 or 6.

2. Compounds according to Claim 1, **characterized in that**
Z1 has the general formula (7) below:

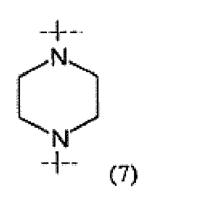

3. Compounds according to Claim 1 or 2, **characterized in that** the compounds have the general formula (9) as shown below, and/or their enantiomers, diastereomers and their pharmaceutically acceptable salts:

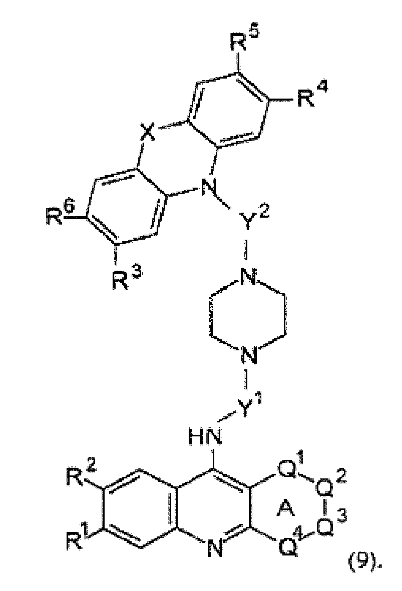

4. Compounds according to any of the preceding claims, **characterized in that**
Q¹, Q³, Q⁴ are each CH and
Q² is selected from the group comprising CH and/or C-O-CH₃.

5. Compounds according to any of the preceding claims, **characterized in that**
Q¹, Q², Q³, Q⁴ are each CH₂.

6. Compounds according to any of the preceding claims, **characterized in that**
Y¹, Y² are each independently of one another selected from the group comprising -(CH₂)ₒ- and/or -CH(CH₃)-(CH₂)ₚ-, wherein:
o is 2, 3, 4, 5 or 6;
p is 2, 3, 4 or 5.

7. Compounds according to any of the preceding claims, **characterized in that**
R¹, R², R³, R⁴, R⁵, R⁶ are each independently of one another selected from the group comprising H, halogen, preferably selected from the group comprising F, Cl and/or Br, NH₂, NHR⁹, wherein the radical R⁹ is selected from the group comprising C₁-C₅-alkyl and/or C₁-C₅-acyl, NO₂, C₁-C₅-alkyl, preferably selected from the group comprising methyl, ethyl, isopropyl and/or tert-butyl, and/or C₁-C₅-alkyloxy, preferably selected from the group comprising -O-methyl, -O-ethyl, -O-isopropyl and/or -0-tert-butyl.

8. Compounds according to any of the preceding claims, **characterized in that**
X is -CH₂-CH₂-.

9. Compounds according to Claim 4, **characterized in that**
Y¹, Y² are each independently of one another selected from the group comprising -(CH₂)ₒ- and/or -CH(CH₃)-(CH₂)ₚ-, wherein:
o is 2, 3, 4, 5 or 6;
p is 2, 3, 4 or 5.

10. Compounds according to Claim 4, **characterized in that**
Y¹, Y² are each independently of one another -(CH₂)ₒ-, wherein:
o is 3, 4 or 5.

11. Compounds according to Claim 10, **characterized in that**
R¹, R², R³, R⁴, R⁵, R⁶ are each independently of one another selected from the group comprising H and/or Cl.

12. Compound according to any of the preceding claims, **characterized by** the formula (10) shown below, and/or its enantiomers, diastereomers and its pharmaceutically acceptable salts:

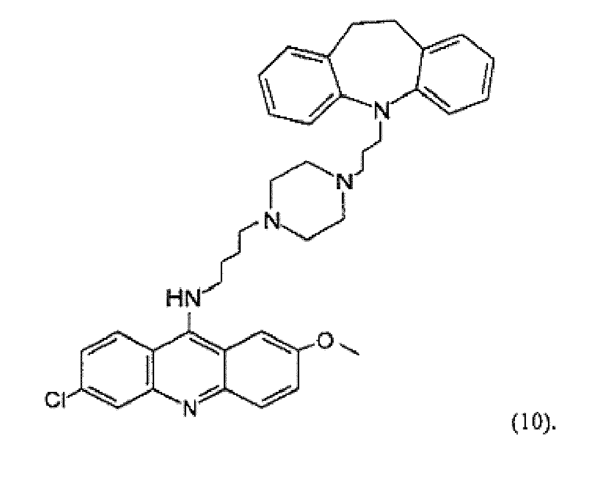

13. Compound according to any of the preceding claims, **characterized by** the formula (14) shown below, and/or its enantiomers, diastereomers and its pharmaceutically acceptable salts:

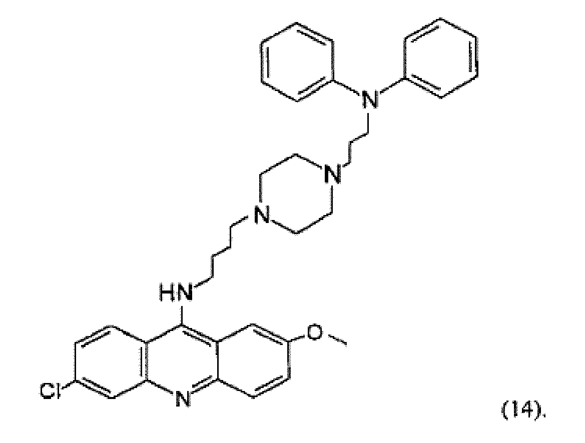

14. Use of compounds according to any of the preceding claims for preparing a medicament.

15. Use of compounds according to any of the preceding claims, preferably of the formula (10), for preparing a medicament for the therapeutic and/or prophylactic treatment of disorders selected from the group comprising:
- disorders associated with protein misfolding, such as prion disorders and/or chronic degenerative disorders, in particular neurodegenerative and/or neuropsychiatric disorders,
- disorders associated with increased blood cholesterol,
- disorders associated with an impaired lipid metabolism and/or hyperlipidaemias,
- disorders associated with an inflammation, such as chronic inflammations,
- cardiovascular disorders, in particular arteriosclerosis, and/or
- infections caused by parasites in animals and plants, in particular humans and useful animals, disorders caused by protozoa and/or by worms, in particular cerebral infections caused by parasites and/or protozoa.

16. Use of compounds according to Claim 15, **characterized in that** the prion disorders are selected from the group comprising Creutzfeldt-Jakob disease, Gerstmann-Straussler-Scheinker disease, fatal familial insomnia, kuru, scrapie and/or bovine spongiform encephalopathy.

17. Use of compounds according to Claim 15, **characterized in that** the chronic degenerative disorders are in particular neurodegenerative and/or neuropsychiatric disorders selected from the group comprising Alzheimer's disease, amyotrophic lateral sclerosis (ALS), Pick's disease, Parkinson's disease, multiple sclerosis, Huntington's disease, diabetes type I and type II, autism, schizophrenia, bipolar disorders, depression, polyglutamine disorders, frontotemporal dementia, tauopathies, multiple system atrophy, sleep disturbances, plasma cell dyscrasia, familial amyloid polyneuropathy, medullary thyroid carcinoma, chronic kidney failure, congestive heart failure, amyloidosis such as cardiac amyloidosis, systemic amyloidosis and/or familial amyloidosis.

18. Use of compounds according to Claim 15, **characterized in that** the infections caused by parasites are selected from the group comprising malaria, trypanosomiasis, sleeping sickness, amoebiasis, Leishmaniasis and/or toxoplasmosis.

19. Medicaments, comprising compounds according to any of the preceding claims, preferably of the formula (10).

20. Antiprion composition, comprising compounds according to any of the preceding claims, preferably of the formula (10).

## Revendications

1. Composés de formule générale (1) telle qu'indiquée ci-après et/ou ses énantiomères, diastéréo-isomères ainsi que ses sels pharmaceutiquement acceptables : 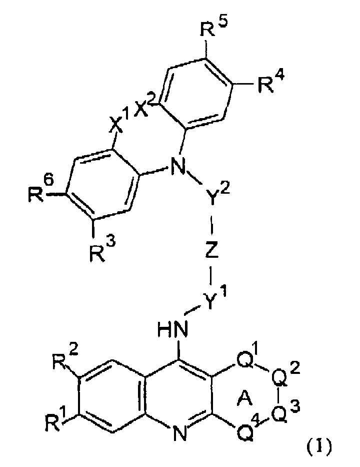 où
A représente un cycle à six chaînons, insaturé ou partiellement saturé ;
Q¹, Q², Q³, Q⁴ sont choisis, à chaque fois indépendamment les uns des autres, dans le groupe comprenant CH, C-halogène, C-O-(C₁-C₁₀)-alkyle, C-CF₃, C-CN et/ou CH₂ ;
R¹, R², R³, R⁴, R⁵, R⁶ sont choisis, à chaque fois indépendamment les uns des autres, dans le groupe comprenant H, halogène, C₁-C₁₀-alkyle, C₁-C₁₀-alcényle, C₁-C₁₀-alcynyle, C₁-C₁₀-alkyloxy, CF₃, NH₂, NHR⁹, le radical R⁹ étant choisi dans le groupe comprenant C₁-C₁₀-alkyle et/ou C₁-C₁₀-acyle, NO₂ et/ou CN ;
X¹, X² représentent à chaque fois H, ou
X¹ et X² forment ensemble X,
X étant choisi dans le groupe comprenant CH₂, CH₂-CH₂, CH=CH et/ou O ;
Y¹, Y² représentent, à chaque fois indépendamment l'un de l'autre, C₁-C₁₀-alkyle non ramifié ou ramifié ;
Z représente un élément de structure Z¹ ou Z² tel qu'indiqué dans la suite :
Z¹ présentant la formule générale (2) ci-après : 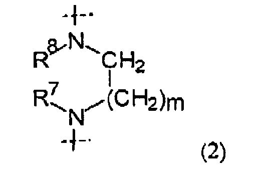 où
m vaut 1, 2, 3, 4, 5 ou 6,
R⁷, R⁸ sont choisis, indépendamment l'un de l'autre, dans le groupe comprenant H, C₁-C₁₀-alkyle, R⁷ et R⁸ formant le cas échéant un cycle via un groupe CH₂-CH₂, et/ou C₁-C₁₀-acyle ;
Z² présentant la formule générale (3) ci-après : 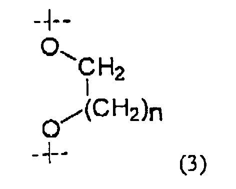 où
n vaut 2, 3, 4, 5 ou 6.

2. Composés selon la revendication 1, **caractérisés en ce que** Z¹ présente la formule générale (7) ci-après :

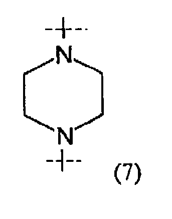

3. Composés selon la revendication 1 ou 2, **caractérisés en ce que** les composés présentent la formule générale (9) telle qu'indiquée ci-après et/ou ses énantiomères, diastéréo-isomères ainsi que ses sels pharmaceutiquement acceptables :

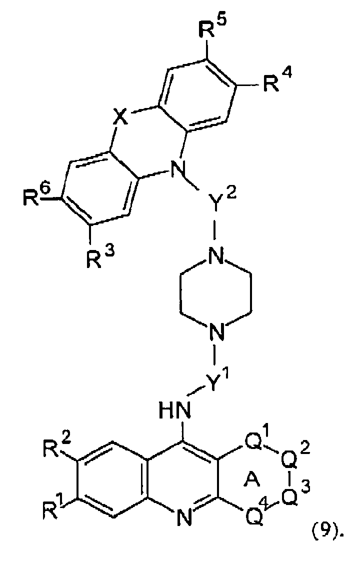

4. Composés selon l'une quelconque des revendications précédentes, **caractérisés en ce que**
Q¹, Q³, Q⁴ représentent à chaque fois CH et
Q² est choisi dans le groupe comprenant CH et/ou C-O-CH₃.

5. Composés selon l'une quelconque des revendications précédentes, **caractérisés en ce que** Q¹, Q², Q³, Q⁴ représentent à chaque fois CH₂.

6. Composés selon l'une quelconque des revendications précédentes, **caractérisés en ce que**
Y¹, Y² sont choisis, à chaque fois indépendamment l'un de l'autre, dans le groupe comprenant -(CH₂)ₒ-et/ou -CH(CH₃)-(CH₂)ₚ- :
o valant 2, 3, 4, 5 ou 6 ;
p valant 2, 3, 4 ou 5.

7. Composés selon l'une quelconque des
revendications précédentes, **caractérisés en ce que**
R¹, R², R³, R⁴, R⁵, R⁶ sont choisis, à chaque fois indépendamment les uns des autres, dans le groupe comprenant H, halogène, de préférence choisi dans le groupe comprenant F, Cl et/ou Br, NH₂, NHR⁹, le radical R⁹ étant choisi dans le groupe comprenant C₁-C₅-alkyle et/ou C₁-C₅-acyle, NO₂, C₁-C₅-alkyle, de préférence choisi dans le groupe comprenant méthyle, éthyle, isopropyle et/ou tert-butyle, et/ou C₁-C₅-alkyloxy, de préférence choisi dans le groupe comprenant -0-méthyle, -O-éthyle, -O-isopropyle et/ou -0-tert-butyle.

8. Composés selon l'une quelconque des revendications précédentes, **caractérisés en ce que**
X représente -CH₂-CH₂-.

9. Composés selon la revendication 4, **caractérisés en ce que**
Y¹, Y² sont choisis, à chaque fois indépendamment l'un de l'autre, dans le groupe comprenant -(CH₂)ₒ-et/ou -CH(CH₃)-(CH₂)ₚ- :
o valant 2, 3, 4, 5 ou 6 ;
p valant 2, 3, 4 ou 5.

10. Composés selon la revendication 4, **caractérisés en ce que**
Y¹, Y² représentent, à chaque fois indépendamment l'un de l'autre, -(CH₂)ₒ-
o valant 3, 4 ou 5.

11. Composés selon la revendication 10,
**caractérisés en ce que**
R¹, R², R³, R⁴, R⁵, R⁶ sont choisis, à chaque fois indépendamment les uns des autres, dans le groupe comprenant H et/ou Cl.

12. Composé selon l'une quelconque des revendications précédentes, **caractérisé par** la formule générale (10) ci-après et/ou ses énantiomères, diastéréo-isomères ainsi que ses sels pharmaceutiquement acceptables :

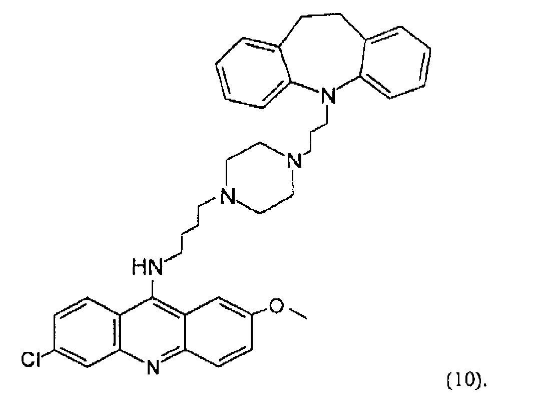

13. Composé selon l'une quelconque des revendications précédentes, **caractérisé par** la formule générale (14) ci-après et/ou ses énantiomères, diastéréo-isomères ainsi que ses sels pharmaceutiquement acceptables :

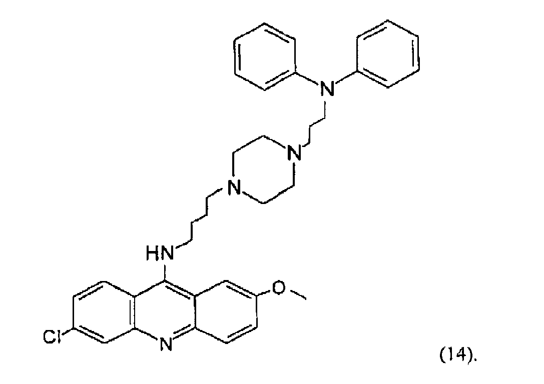

14. Utilisation de composés selon l'une quelconque des revendications précédentes pour la préparation d'un médicament.

15. Utilisation de composés selon l'une quelconque des revendications précédentes, de préférence de formule (10), pour la préparation d'un médicament pour le traitement thérapeutique et/ou prophylactique de maladies, choisies dans le groupe comprenant :
- les maladies qui sont associées à un repliement erroné des protéines, telles que les maladies à prions et/ou les maladies chroniques-dégénératives, en particulier les maladies neurodégénératives et/ou neuropsychiatriques,
- les maladies qui sont associées à une augmentation du cholestérol dans le sang,
- les maladies qui sont associées à un trouble du métabolisme des lipides et/ou les hyperlipidémies,
- les maladies qui sont associées à une inflammation, telles que les inflammations chroniques,
- les maladies cardiovasculaires, en particulier l'artériosclérose, et/ou
- les infections provoquées par des parasites chez les animaux et les plantes, en particulier les hommes et les animaux utiles, les maladies provoquées par les protozoaires et/ou les vers, en particulier les infections cérébrales provoquées par les parasites et/ou les protozoaires.

16. Utilisation de composés selon la revendication 15, **caractérisée en ce que** les maladies à prions sont choisies dans le groupe comprenant la maladie de Creutzfeldt-Jakob, la maladie de Gerstmann-Sträussler-Scheinker, l'insomnie fatale familiale, le kuru, la scrapie et/ou l'encéphalopathie spongiforme bovine.

17. Utilisation de composés selon la revendication 15, **caractérisée en ce que** les maladies chroniques-dégénératives, en particulier les maladies neurodégénératives et/ou neuropsychiatriques, sont en particulier choisies dans le groupe comprenant la maladie d'Alzheimer, la sclérose amyotrophique latérale (SAL), la maladie de Pick, la maladie de Parkinson, la sclérose en plaques, la maladie d'Huntington, le diabète de type I et de type II, l'autisme, la schizophrénie, les maladies bipolaires, la dépression, les maladies à polyglutamine, la démence frontotemporale, les tauopathies, l'atrophie multisystématisée, les troubles du sommeil, la dyscrasie plasmocytaire, la polyneuropathie amyloïde familiale, le carcinome médullaire de la thyroïde, l'insuffisance rénale chronique, l'insuffisance cardiaque congestive, l'amyloïdose telle que l'amyloïdose cardiaque, l'amyloïdose systémique et/ou l'amyloïdose familiale.

18. Utilisation de composés selon la revendication 15, **caractérisée en ce que** les infections provoquées par des parasites sont choisies dans le groupe comprenant la malaria, la trypanosomiase, la maladie du sommeil, la dysenterie amibienne, la leishmaniose et/ou la toxoplasmose.

19. Médicament comprenant des composés selon l'une quelconque des revendications précédentes, de préférence de formule (10).

20. Agent anti-prion comprenant des composés selon l'une quelconque des revendications précédentes, de préférence de formule (10).
